(19) Europäisches Patentamt
European Patent Office
Office européen des brevets

(11) EP 1 763 383 B1

(12) **EUROPÄISCHE PATENTSCHRIFT**

(45) Veröffentlichungstag und Bekanntmachung des Hinweises auf die Patenterteilung:
**23.12.2009 Patentblatt 2009/52**

(21) Anmeldenummer: **05744684.1**

(22) Anmeldetag: **12.05.2005**

(51) Int Cl.:
***A61K 8/11*** *(2006.01)* ***A61Q 17/02*** *(2006.01)*
***A61Q 17/04*** *(2006.01)*

(86) Internationale Anmeldenummer:
**PCT/EP2005/005179**

(87) Internationale Veröffentlichungsnummer:
**WO 2005/120440 (22.12.2005 Gazette 2005/51)**

(54) **PARTIKEL, FUNKTIONALISIERT MIT ORGANISCHEN VERBINDUNGEN**

PARTICLE FUNCTIONALISED WITH ORGANIC COMPOUNDS

PARTICULE FONCTIONNALISEE AVEC DES COMPOSES ORGANIQUES

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **08.06.2004 EP 04013515**

(43) Veröffentlichungstag der Anmeldung:
**21.03.2007 Patentblatt 2007/12**

(73) Patentinhaber: **Merck Patent GmbH**
**64293 Darmstadt (DE)**

(72) Erfinder:
- **WALENZYK, Thomas**
  **65830 Kriftel (DE)**
- **CAROLA, Christophe**
  **69120 Heidelberg (DE)**
- **BUCHHOLZ, Herwig**
  **60599 Frankfurt (DE)**

(56) Entgegenhaltungen:
**US-A1- 2002 150 600 US-B1- 6 238 650**

- **PANDEY S ET AL: "On the microenvironments surrounding dansyl sequestered within class I and II xerogels" CHEMISTRY OF MATERIALS, AMERICAN CHEMICAL SOCIETY, WASHINGTON, US, Bd. 12, 2000, Seiten 3547-3551, XP002327240 ISSN: 0897-4756**
- **SPANGE S ET AL: "A one-pot synthesis of chromophoric silicate-based xerogels" ANGEWANDTE CHEMIE, WILEY-VCH, WEINHEIM, DE, Bd. 41, Nr. 10, 2002, Seiten 1729-1732, XP002327219 ISSN: 1433-7851**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 763 383 B1

Printed by Jouve, 75001 PARIS (FR)

## Beschreibung

Die vorliegende Erfindung betrifft Partikel gemäß Anspruch 1 umfassend ein anorganisches Netzwerk und über eine Spacer-Gruppe kovalent an das Netzwerk gebundene organische Verbindungen, wobei die organischen Verbindungen im Inneren der Partikel und auf der Oberfläche der Partikel vorliegen. Weiterhin betrifft die vorliegende Erfindung Verfahren zur Herstellung der Partikel sowie deren Verwendung in Formulierungen und Zubereitungen, insbesondere in Zubereitungen mit Lichtschutzeigenschaften.

Organische Verbindungen spielen im Zusammenhang mit kosmetischen oder dermatologischen Formulierungen und Zubereitungen eine große Rolle. So werden beispielsweise organische Verbindungen unterschiedlichster Verbindungsklassen als UV-Filter eingesetzt, um die Haut vor den Einwirkungen des Sonnenlichts und damit verbundenen unerwünschten Nebenwirkungen, wie z.B. Hautalterung oder Faltenbildung, zu schützen. Üblicherweise wirken die gängigen UV-Filter, indem sie einen Film auf der Oberfläche der Haut bilden. Die UV-Filter absorbieren bestimmte Bereiche des Sonnenlichts, so dass die gefilterte Strahlung nicht in tiefere Schichten der Haut eindringen kann. Gleichzeitig wird aber zunehmend versucht, das Eindringen der UV-Filter in die Haut ebenfalls zu unterbinden, um mögliche Hautschädigungen oder allergieauslösende Prozesse zu verhindern.

So wird in EP 1 205 177 vorgeschlagen, ein Konjugat umfassend ein organisches Pigment und einen Wirkstoff auf der Basis organischer Verbindungen, z.B. einen UV-Filter, der kovalent an das anorganische Pigment gebunden ist, einzusetzen. Zur Herstellung dieses Konjugats werden entsprechend funktionalisierte organische Verbindungen an der Oberfläche der Pigmente angebunden. Die auf diese Weise erhaltenen UV-Filter/Pigment-Systeme können aufgrund ihres partikulären Charakters nicht in die Haut eindringen. Der Kontakt der Haut mit den auf der Oberfläche der Pigmente aufgebrachten Verbindungen wird auf diese Weise aber nicht gänzlich vermieden, da die Verbindungen auf der Oberfläche in Wechselwirkung mit der Haut treten können. Weiterhin ist der maximal aufzubringende Anteil an organischen Verbindungen durch die Oberfläche des Pigmentes begrenzt, das heißt die maximale Wirkstoffkonzentration korreliert direkt mit der zur Verfügung stehenden Oberfläche. Gerade im Bereich der UV-Filter ist ein möglichst hoher Anteil an Wirkstoff besonders wichtig, um eine maximale Schutzwirkung zu erzielen, das heißt, um einen optimalen Schutz vor der Sonnenstrahlung zu gewährleisten. Es besteht daher ein dringender Bedarf an Wirkstoff/TrägerSystemen, die zum einen nur eine geringe Tendenz zur Penetration in die Haut aufweisen und zum anderen einen möglichst hohen Wirkstoffanteit aufweisen.

Der vorliegenden Erfindung lag demgemäss die Aufgabe zugrunde, Materialien bereit zu stellen, die die vorteilhaften Wirkungen, vorzugsweise lichtabsorbierenden Wirkungen, organischer Verbindungen besser nutzbar machen lassen, ohne dass ein Eindringen der organischen Verbindungen in die Haut vonnöten wäre.

Diese Aufgabe wird durch die Partikel gemäß der vorliegenden Erfindung gelöst. Gegenstand der vorliegenden Erfindung sind demgemäss Partikel umfassend ein anorganisches Netzwerk und über eine Spacer-Gruppe kovalent an das Netzwerk gebundene organische Verbindungen, wobei die organischen Verbindungen im Inneren der Partikel und auf der Oberfläche der Partikel vorliegen. Weiterer Gegenstand der vorliegenden Erfindung sind Verfahren zur Herstellung der erfindungsgemäßen Partikel, umfassend

a) das Umsetzen einer organischen Verbindung unter Ausbildung einer kovalenten Bindung mit einer Substanz mit mindestens zwei durch eine Spacer-Gruppe getrennten reaktiven Gruppen und
b) die Co-Polymerisation der in a) erhaltenen Verbindung mit einem für den Aufbau eines anorganischen Netzwerks geeigneten Precursor.

Die Verwendung der erfindungsgemäßen Partikel in Formulierungen und Zubereitungen sowie Zubereitungen enthaltend die erfindungsgemäßen Partikel ist ebenfalls Gegenstand der vorliegenden Erfindung.

Die Partikel gemäß der vorliegenden Erfindung haben den Vorteil, das durch die Einbindung der organischen Verbindungen in den Kern der Partikel der Kontakt z.B. mit der Haut minimiert wird. Darüber hinaus lassen sich bei einer Mehrzahl der geeigneten Partikel durch das im Verhältnis zur Oberfläche größere Volumen der Partikel mehr Wirkstoffe in den Partikeln integrieren als dies mit einer reinen Aufbringung auf der Oberfläche möglich wäre. Ein weiterer Vorteil ist, dass durch die Einbindung der organischen Verbindungen in den Kern der Partikel Zubereitungsprobleme bei der Herstellung von Formulierungen, die durch Wechselwirkung einzelner Zubereitungsbestandteile untereinander entstehen, wie Kristallisationsvorgänge, Ausfällungen und Agglomeratbildung, vermieden werden. Darüber hinaus kann sich die Photostabilität der eingesetzten organischen Verbindungen, insbesondere bei UV-Filtem, erhöhen, wenn sie, wie bei der vorliegenden Erfindung, in den Kern einer Matrix eingebettet vorliegen.

Die erfindungsgemäßen Partikel umfassen ein anorganisches Netzwerk und über eine Spacer-Gruppe kovalent an das Netzwerk gebundene organische Verbindungen, wobei die Verbindungen im Kern der Partikel und auf der Oberfläche der Partikel vorliegen. Die Form der Partikel ist an sich nicht kritisch, vorzugsweise handelt es sich um sphärische Partikel. Nicht-funktionalisierte sphärische Partikel lassen sich besonders einfach herstellen und zeigen, z.B. bei Aufbringung auf die Haut, ein besonders gutes Hautgefühl. Dagegen erweist sich die Herstellung auf der

Oberfläche funktionalisierter sphärischer Partikel als schwierig, da durch die Funktionalisierung eine Vernetzung der sich bildenden Partikel auftreten kann, die die Ausbildung von Sphären erschwert oder ganz verhindert. Die vorliegende Erfindung erlaubt daher die Herstellung von sphärischen Partikeln mit einer im Kern der Partikel vorliegenden Funktionalisierung, die die vorteilhaften Eigenschaften der Sphären, z.B. besseres Hautgefühl, mit den vorteilhaften Eigenschaften der organischen Verbindungen, z.B. UV-Schutz, kombiniert. Die Größe, im einfachsten Falle der maximale Durchmesser, der Partikel, kann zwischen 1 nm und 250 μm variieren, vorzugsweise liegt sie im Bereich von 1 nm bis 1 μm und ganz besonders bevorzugt zwischen 30 nm und 700 nm.

Das anorganische Netzwerk der erfindungsgemäßen Partikel ist ein Oxid, Oxidhydrat, Phosphat, Carbonat, Sulfat oder Nitrid sein, wobei das Oxid, Oxidhydrat, Phosphat, Carbonat, Sulfat oder Nitrid eines Metalls, Halbmetalls oder Nichtmetalls vorliegen kann, vorzugsweise handelt es sich um ein Oxid eines Metalls oder Halbmetalls. Entsprechende Oxide sind beispielsweise Magnesiumoxid, Aluminiumoxyd, Siliziumoxid, Zinkoxid, Ceroxid, Titanoxid, Zirkoniumoxid, Manganoxid, Boroxid, Eisenoxid oder ein Gemisch hieraus. Beispiele für Phosphate, Carbonate, Sulfate oder Nitride sind unter anderem Magnesiumcarbonat, Calciumcarbonat, Bariumsulfat, Calciumsulfat, Calciumphosphat, Bornitrid, wobei die genannten Stoffe einzeln oder als Gemisch vorliegen können. Vorzugsweise handelt es sich bei dem anorganischen Netzwerk um Siliziumoxid, insbesondere um Siliziumdioxid. Partikel, insbesondere sphärische Partikel, enthaltend Siliziumoxid als anorganisches Netzwerk sind gut zugänglich und erweisen sich als besonders gut geeignet in dermatologischen Anwendungen, da sie mit der Haut nicht reagieren, chemisch weitestgehend inert und damit toxikologisch unbedenklich sind. Im Falle von sphärischen $SiO_2$-Partikeln kann bei Anwendung in topischen Zubereitungen ein gutes Hautgefühl erzeugt werden, was auf den sphärischen Charakter der Partikel zurückgeführt werden kann.

Die kovalent an das Netzwerk gebundenen organischen Verbindungen sind ausgewählt aus der Gruppe der lichtabsorbierenden organischen Verbindungen, der Verbindungen mit antioxidanten und/oder radikalinhibierenden Eigenschaften, der Repellentien, der Konservierungsmittel und/oder Derivate dieser Verbindungen, vorzugsweise handelt es sich um lichtabsorbierende Verbindungen.

Die lichtabsorbierenden Verbindungen sind ausgewählt aus der Gruppe aus Dibenzoylmethanderivaten, Aminobenzoesäure, Zimtsäure, Salicylsäure, Benzylidenkampfer, Phenylbenzimidazol, Diphenylacrylat, Triazin, Benzophenon, Diarylbutadien, vinylgruppenhaltigen Amiden und/oder Derivaten davon, die beispielsweise in der Anmeldung WO 93/04665 beschrieben sind. Weitere Beispiele für organische Filter sind in der Patentanmeldung EP-A 0 487 404 angegeben.

Die erfindungsgemäß verwendbaren Dibenzoylmethanderivate können insbesondere unter den Dibenzoylmethanderivaten der folgenden Formel ausgewählt sein:

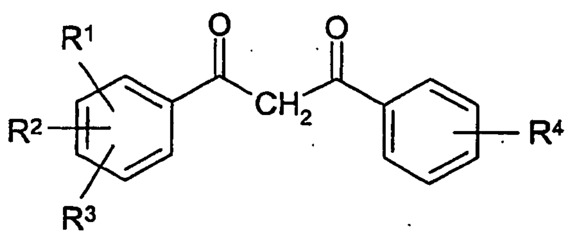

worin $R^1$, $R^2$, $R^3$ und $R^4$, die identisch oder voneinander verschieden sind, Wasserstoff, eine geradkettige oder verzweigte $C_{1-8}$-Alkylgruppe oder eine geradkettige oder verzweigte $C_{1-8}$-Alkoxygruppe bedeuten. Gemäß der vorliegenden Erfindung können selbstverständlich ein Dibenzoylmethanderivat oder mehrere Dibenzoylmethanderivate verwendet werden. Von den Dibenzoylmethanderivaten, auf die sich die vorliegende Erfindung spezieller bezieht, können insbesondere:

- 2-Methyldibenzoylmethan,
- 4-Methyldibenzoylmethan,
- 4-Isopropyldigenzoylmethan,
- 4-tert.-Butyldibenzoylmethan,
- 2,4-Dimethyldibenzoylmethan,
- 2,5-Dimethyldibenzöylmethan,
- 4,4'-Diisopropyldibenzoylmethan,
- 4,4'-Methoxy-tert.-butyldibenzoylmethan,
- 2-Methyl-5-isopropyl-4'-methoxydibenzoylmethan,
- 2-Methyl-5-tert.-butyl-4'-methoxydibenzoylmethan,
- 2,4-Dimethyl-4'-methoxydibenzoylmethan
  und 2,6-Dimethyl-4-tert.-butyl-4'-methoxydibenzoylmethan genannt werden,

wobei diese Aufzählung nicht einschränkend ist. Von den obengenannten Dibenzoylmethanderivaten wird erfindungsgemäß insbesondere das 4,4'-Methoxy-tert.-butyldibenzoylmethan und insbesondere das unter der Handelsbezeichnung Eusolex® 9020 von der Firma Merck KGaA im Handel befindliche 4,4'-Methoxy-tert.-butyldibenzoylmethan bevorzugt, wobei dieses Filter der folgenden Strukturformel entspricht:

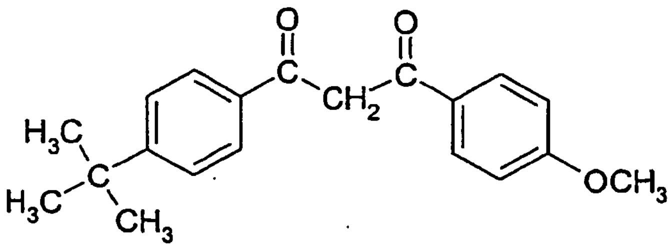

Ein weiteres erfindungsgemäß bevorzugtes Dibenzoylmethanderivat ist das 4-Isopropyldibenzoylmethan.

Weiterhin kann es sich bei den lichtabsorbierenden Verbindungen um Benzophenon oder Derivate des Benzophenons handeln, wie insbesondere bevorzugt 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40). Weitere Beispiele geeigneter lichtabsorbierender Verbindungen umfassen Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl] benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL), Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000), Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS), 4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25), Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4.,6-disulfonsäure bzw. deren Salze (z.B. Neoheliopan® AP) oder 2,2-(1,4-Phenylen)-bisbenzimidazol-6-sulfonsäure, und weitere Substanzen wie

- 2-Cyano-3,3-diphenylacrylsäure-2-ethylhexylester (z.B. Eusolex® OCR),
- 3,3'-(1,4-Phenylendimethylen)-bis-7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin ( z.B. Uvinul® T 150)
- 2-(4-Diethylamino-2-hydroxy-benzoyl)-benzoesäure hexylester (z.B. Uvinul®UVA Plus, Fa. BASF).

Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- α-(Trimethylsilyl)-ω-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5 % methyl[3-[p-[2,2-bis(ethoxycarbonyl)vinyl)phenoxy)propenyl) und 0,1 bis 0,4% (methylhydrogen]silylen]] (n≈ 60) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Bevorzugte Verbindungen mit UV-filtemden Eigenschaften sind 3-(4'-Methylbenzyliden)-dl-kampfer, 1-(4-tert-Butylphenyl)-3-(4-methoxy-phenyl)-pro-pan-1,3-dion, 4-lsopropyldibenzoylmethan, 2-Hydroxy-4-meth-oxybenzophenon, Methoxyzimtsäureoctylester, 3,3,5-Trimethyl-cyclo-hexylsali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanolaminsalze.

Ganz besonders bevorzugt eingesetzt werden 2-Hydroxy-4-methoxybenzophenon und/oder 4,4'-Methoxy-tert.-butyldibenzoylmethan.

Geeignete Substanzen mit antioxidanten und/oder radikalinhibierenden Eigenschaften umfassen z.B. Flavonoide, Coumarone, Aminosäuren (z.B. Glycin, Histidin, Tyrosin, Tryptophan) und deren Derivate, Imidazole (z.B. Urocaninsäure) und deren Derivate, Peptide, wie z.B. D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogonsäure, und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acyl-, Methyl-, Ethyl-, Propyl-, Amyl-, Butyl- und Lauryl-, Palmitoyl-, Oleyl-, Linoleyl-, Cholesteryl- und Glycerylester), Diaurylthiodipropionat, Distearylthiodipropionat, Thiodiproiosäure und deren Derivate (Ester, Ether, Peptide, Lipide, Nukleotide und Nukleoside) sowie Sulfoximverbindungen (z.B. Buthioninsulfoxime, Homocysteinsulfoxim, Buthioninsulfone, Penta-, Hexa-, Heptathioninsulfoximin), Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Ascorbylacetät), Toco-pherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Vitamin E und dessen Derivate, Stilbene und deren Derivate (z.B. Stilbenoxid, trans-Stilbenoxid) sowie BHT (2,6-Di-tert-Butyl-4-methyl-phenol).

Bevorzugte Antioxidantien umfassen Flavonoide, Coumaranone, Vitamine und BHT.

Als Flavanoide werden die Glycoside von Flavanonen, Flavonen, 3-Hydroxyflavonen (= Flavanolen), Auronen, Isoflavonen und Rotenoiden aufgefaßt (Römpp Chemie Lexikon, Band 9, 1993). Im Rahmen der vorliegenden Erfindung werden hierunter jedoch auch die Aglykone, d.h. die zuckerfreien Bestandteile, und die Derivate der Flavonoide und der Aglykone verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und Isoflavonen, insbesondere von Flavanonen, Flavonen, 3-Hydroxyflavonen und Auronen, ab. Vorzugsweise werden die Flavonoide und Coumaranone ausgewählt aus den Verbindungen der Formel (1):

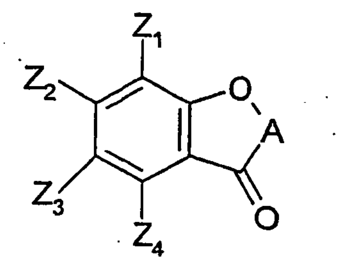

(1)

worin bedeuten:

$Z_1$ bis $Z_4$ jeweils unabhängig voneinander H, OH, Alkoxy (OR), Hydroxyalkoxy, Mono- oder Oligoglycosidreste, wobei die Alkoxy- und Hydroxyalkoxygruppen verzweigt und unverzweigt sein und 1 bis 18 Kohlenstoffatome aufweisen können und wobei an die Hydroxygruppen der genannten Reste auch Sulfat oder Phosphat gebunden sein kann,

A ausgewählt wird aus der Gruppe, bestehend aus den Teilformen (1A), (1B) und (1C)

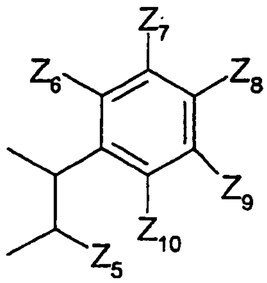

(1A)

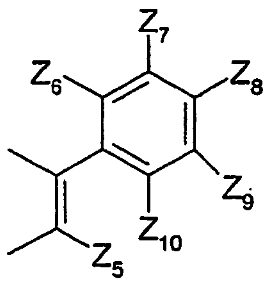

(1B)

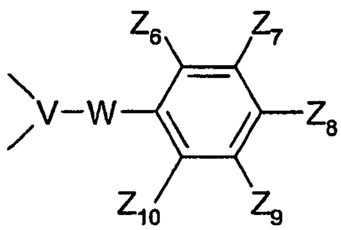

(1C)

mit $Z_5$ H, OH oder OR,
$Z_6$ bis $Z_{10}$ die Bedeutung der Reste $Z_1$ bis $Z_4$ besitzen, und

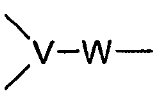 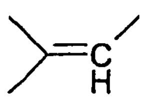 oder 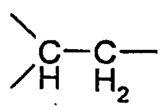

bedeutet.

Die Alkoxygruppen sind vorzugsweise linear und besitzen 1 bis 12, vorzugsweise 1 bis 8 Kohlenstoffatome. Diese Gruppen entsprechen somit der Formel $-O-(CH_2)_m-H$, wobei m 1,2,3,4,5,6,7 oder 8 und insbesondere 1 bis 5 bedeutet.

Die Hydroxyalkoxygruppen sind vorzugsweise linear und besitzen 2 bis 12, vorzugsweise 2 bis 8 Kohlenstoffatome. Diese Gruppen entsprechen somit der Formel $-O-(CH_2)_n-OH$, wobei n 2,3,4,5,6,7 oder 8, insbesondere 2 bis 5 und besonders bevorzugt 2 bedeutet.

Die Mono- und Oligoglycosidreste sind vorzugsweise aus 1 bis 3 Glycosideinheiten aufgebaut. Vorzugsweise werden diese Einheiten ausgewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl, sind gegebenenfalls vorteilhaft zu verwenden. Es kann auch erfindungsgemäß vorteilhaft sein, Pentosylreste zu verwenden.

In einer bevorzugten Ausführungsform besitzen
$Z_1$ und $Z_3$ die Bedeutung H,
$Z_2$ und $Z_4$ eine andere Bedeutung als H, insbesondere bedeuten sie
OH, Methoxy, Ethoxy oder 2-Hydroxyethoxy,
$Z_5$ die Bedeutung H, OH oder einen Glycosidrest, der aus 1 bis 3, vorzugsweise aus 1 oder 2, Glycosideinheiten aufgebaut ist.
$Z_6$, $Z_9$ und $Z_{10}$ die Bedeutung H, und
$Z_7$ und $Z_8$ eine andere Bedeutung als H, insbesondere bedeuten sie OH, Methoxy, Ethoxy oder 2-Hydroxyethoxy.

In einer weiteren bevorzugten Ausführungsform werden die Flavonoide ausgewählt aus folgenden Verbindungen: 4,6,3',4'-Tetrahydroxyauron, Quercetin, Rutin, Isoquercetin, Anthocyanidin (Cyanidin), Eriodictyol, Taxifolin, Luteolin, Trishydroxyethylquercetin (Troxequercetin), Trishydroxyethylrutin (Troxerutin), Trishydroxyethylisoquercetin (Troxeisoquercetin) sowie Trishydroxyethylluteolin (Troxeluteolin). Unter den Flavonoiden sind insbesondere Rutin und Troxerutin bevorzugt. Besonders bevorzugt ist Troxerutin. Unter den Coumaranonen ist 4,6,3',4'-Tetrahydroxybenzylcoumaranon-3 bevorzugt. Weitere geeignete Antioxidantien sind weiter unten genannt bzw. sind dem Fachmann ohne erfinderisches Zutun zugänglich.

Geeignete Repellentien umfassen Amide und deren Derivate, insbesondere N,N-Diethyl-3-methyl-benzamid, 3-[N-n-Butyl-N-acetyl]-aminopropionsäureethylester (IR3535®) und N,N-Caprylsäurediethylamid (IR790®).

Geeignete Konservierungsmittel umfassen Benzalkoniumchlorid, Benzoesäure und deren Salze (wie z.B. Natriumbenzoat), Methylparaben, Ethylparaben, Propylparaben, Sorbinsäure und deren Salze (wie z.B. Kaliumsorbat), Cetylpyridiniumchlorid, Cetrimoniumchlorid sowie Salicylsäure und deren Salze (wie z.B. Natriumsalicylat).

Die Spacer-Gruppe, die das anorganische Netzwerk mit der organischen Verbindung verknüpft, hat die allgemeine Formel

$$(-A)_aMe(R^1)_b(R^2)_c\text{-}X\text{-}$$

wobei
A = O, S, NH bedeutet
Me = Si, Al, Ti, Zr bedeutet
$R^1$ und $R^2$ gleich oder verschieden sein können und eine geradkettige oder verzweigte Alkyl-, Alkoxy-, Halogen- oder Hydroxygruppe bedeuten a 1 bis 3 ist
b und c 0-2 sein können, mit der Bedingung, dass für Me = Si, Ti, Zr a+b+c=3 ist und für Me = Al a+b+c=2 ist und
X = eine geradkettige oder verzweigte Alkylgruppe mit bis zu 20, vorzugsweise mit 1 bis 12 und besonders bevorzugt mit 3 bis 12 Kohlenstoffatomen bedeutet, und wobei die Spacer-Gruppe über den Substituenten $(-A)_a$ an das anorganische Netzwerk gebunden ist.

Über das Zentralatom Me der Spacer-Gruppe können in Abhängigkeit der Anzahl der Substituenten (-A) eine oder mehrere kovalente Bindungen zum anorganischen Netzwerk vorliegen. Vorzugsweise handelt es sich bei dem Zentralatom Me um Silicium. Spacer-Gruppen dieser Art eignen sich in besonderer Weise zur Verknüpfung organischer Verbindungen mit dem anorganischen Netzwerk.

Die geradkettige oder verzweigte $C_1$-$C_{20}$-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl, Dodecyl, Tridecyl oder Tetradecyl. Gegebenenfalls perfluorierte Alkylgruppen, beispielsweise Difluormethyl, Trifluormethyl, Pentafluorethyl, Heptafluorpropyl oder Nonafluorbutyl. Bevorzugt ist die geradkettige oder verzweigte Alkylgruppe Isopropyl, Propyl, Butyl.

Die geradkettige oder verzweigte Alkoxygruppe ist beispielsweise Methoxy, Ethoxy, Propoxy, Butoxy, Pentoxy, Isopropoxy, vorzugsweise Methoxy oder Ethoxy.

Halogen ist F, Cl, Br, I, vorzugsweise Cl oder Br.

Zur Funktionalisierung der Partikel liegen organische Verbindungen an der Oberfläche kovalent gebunden vor. Die organischen Verbindungen können aus der Gruppe der lichtabsorbierenden organischen Verbindungen, der Verbindungen mit antioxidanten und/oder radikalinhibierenden Eigenschaften, der Repellentien, der Konservierungsmittel ausgewählt sein. Beispiele für die oben genannten Verbindungen sind vorab bei der Beschreibung der erfindungsgemäßen Partikel genannt. Der Anteil der organischen Verbindungen und damit beispielsweise der Wirkstoffe kann auf diese Weise weiter erhöht werden. Im Falle von z.B. UV-Filtern kann der Anteil an absorbierender Substanz pro Partikel gesteigert werden, um die schützende Wirkung der UV-Filter verstärken zu können. Die Oberfläche der Partikel kann mit den identischen, der im Inneren der Partikel vorliegenden organischen Verbindungen, und/oder davon verschiedenen organischen Verbindungen funktionalisiert sein. Damit ist auch eine Kombination unterschiedlicher Wirkstoffe realisierbar, das heißt, dass sich beispielsweise im Inneren der Partikel ein UV-Filter und auf der Oberfläche Verbindungen mit antioxidanten Eigenschaften oder Repellentien befinden. Für die einzelnen Kombinationen gibt es dabei keine Einschränkungen, das heißt alle Wirkstoffe lassen sich miteinander kombinieren, so dass multifunktionelle Partikel erhalten

werden.

Ebenfalls Gegenstand der vorliegenden Erfindung sind Verfahren zur Herstellung der erfindungsgemäßen Partikel gemäß Anspruch 10 umfassend

a) das Umsetzen einer organischen Verbindung unter Ausbildung einer kovalenten Bindung mit einer Substanz mit mindestens zwei durch eine Spacer-Gruppe getrennten reaktiven Gruppen und
b) die Co-Polymerisation der in a) erhaltenen Verbindung mit einem für den Aufbau eines anorganischen Netzwerks geeigneten Precursor.

Im ersten Schritt des erfindungsgemäßen Verfahrens wird eine organische Verbindung mit einer Substanz mit mindestens zwei durch eine Spacer-Gruppe getrennten reaktiven Gruppen umgesetzt, so dass die organische Verbindung kovalent an die Substanz gebunden wird. Als Substanzen mit mindestens zwei durch eine Spacer-Gruppe getrennten reaktiven Gruppen können jene gemäß der allgemeinen Formel

$$(R^3A)_aMe(R^1)_b(R^2)_c\text{-X-Y}$$

eingesetzt werden, wobei
A = O, S, NH bedeutet
Me = Si, Al, Ti, Zr bedeutet
$R^1$ und $R^2$ gleich oder verschieden sein können und eine geradkettige oder verzweigte Alkyl-, Alkoxy-, Halogen-, oder Hydroxygruppe bedeuten
$R^3$ = Wasserstoff oder eine geradkettige oder verzweigte Alkylgruppe bedeutet a = 1 bis 3 ist
b und c 0-2 sein können, mit der Bedingung, dass für Me = Si, Ti, Zr $a+_b+c=3$ ist und für Me = Al a+b+c=2 ist, und
X = eine geradkettige oder verzweigte Alkylgruppe mit bis zu 20, vorzugsweise mit 1 bis 12 und besonders bevorzugt mit 3 bis 12 Kohlenstoffatomen bedeutet, und
Y = geradkettiges oder verzweigtes Alkenyl, Alkinyl, Alkoxy, SH, $NH_2$, Carboxy, Halogen, oder Hydroxy bedeutet, wobei die kovalente Bindung zwischen der organischen Verbindung und der Spacer-Gruppe unter Abspaltung oder über Verknüpfung mit der reaktiven Gruppe Y ausgebildet wird. Generell eignen sich für die Verknüpfung alle dem Fachmanns bekannten Reaktionstypen zur Herstellung kovalenter Bindungen, wie z.B: die Addition, Substitution, Hydrosilylierung etc. Die Auswahl geeigneter Verknüpfungsreaktionen unterliegt dem Fachwissen des Fachmanns und findet sich beispielsweise in EP 1 205 177.

Ein geradkettiges oder verzweigtes Alkenyl mit 2 bis 20 C-Atomen, wobei auch mehrere Doppelbindungen vorhanden sein können, ist beispielsweise Vinyl, Allyl, 2- oder 3-Butenyl, Isobutenyl, sek.-Butenyl, ferner 4-Pentenyl, iso-Pentenyl, Hexenyl, Heptenyl, Octenyl, $-C_9H_{17}$, $-C_{10}H_{19}$ bis $-C_{20}H_{39}$; vorzugsweise Vinyl, Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, $-C_9H_{15}$, $-C_{10}H_{17}$ bis $-C_{20}H_{37}$, vorzugsweise Ethinyl, 1- oder 2-Propinyl, 2- oder 3-Butinyl, 4-Pentinyl, 3-Pentinyl oder Hexinyl.

Die organischen Verbindungen, die mit der Spacer-Gruppe verknüpft werden, können ausgewählt sein aus der Gruppe der lichtabsorbierenden organischen Verbindungen, der Verbindungen mit antioxidanten und/oder radikalinhibierenden Eigenschaften, der Repellentien, der Konservierungsmittel. Beispiele hierfür sind bereits genannt.
Bei dem zweiten Schritt der erfindungsgemäßen Verfahren erfolgt die Co-Polymerisation der im ersten Schritt erhaltenen Verbindung mit einem für den Aufbau eines anorganischen Netzwerks geeigneten Precursor. Vorzugsweise handelt es sich bei dem zweiten Schritt um eine hydrolytische Polykondensation. Auf diese Weise lassen sich eine Vielzahl von anorganischen Netzwerken aufbauen, wobei die Reaktionsbedingungen in vielfältiger Weise angepasst werden können. Verfahren dieser Art sind bekannt und finden sich beispielsweise in den Publikationen von W. STÖBER et al. in J. Colloid and Interface Science 26, 62 (1968) und 30, 568 (1969) sowie dem US Patent 3,634,588, denen die grundlegenden Reaktionsbedingungen hierfür zu entnehmen sind. Diese sehen vor, dass beispielsweise Tetraalkoxysilan in einen Überschuss eines wässrigalkoholisch-ammoniakalischen Hydrolysegemisches gebracht wird, wobei durch geeignete Maßnahmen wie Rühren, Schütteln oder Ultraschallbehandlung für eine intensive Durchmischung gesorgt wird. Hierbei lassen sich je nach Wahl der konkreten experimentellen Parameter $SiO_2$-Partikel unterschiedlicher mittlerer Teilchengröße und variierender Teilchengrößenverteilung erhalten. Dieses sowie analoge Verfahren eignen sich zur Herstellung der anorganischen Netzwerke gemäß der vorliegenden Erfindung.

Als Precursor für den Aufbau des anorganischen Netzwerks können beispielsweise Hydroxide, Halogenide, Alkoxide oder andere hydrolisierbare Gruppen enthaltende Verbindungen der Elemente Magnesium, Aluminium, Silizium, Zink, Cer, Titan, Zirkonium, Mangan, Bor, Eisen und/oder Gemische hieraus eingesetzt werden. Vorzugsweise handelt es sich bei dem Precursor um Alkoxide des Siliziums, wie z.B. Tetraalkoxysilane. Als geeignete Tetraalkoxysilane

können alle problemlos hydrolysierbaren Kieselsäureorthoester aliphatischer Alkohole eingesetzt werden. In erster Linie kommen hierbei die Ester aliphatischer Alkohole mit 1 bis 5 C-Atomen in Betracht, das heißt die Alkoxygruppe kann eine Methoxy-, Ethoxy-, Propoxy-, Butoxy- oder Pentoxygruppe sein, wobei die jeweiligen Isomeren berücksichtigt sind. Die oben genannten Tetraalkoxysilane können einzeln, aber auch im Gemisch eingesetzt werden. Bevorzugt eingesetzt werden die Kieselsäureorthoester der $C_1$ -$C_3$-Alkohole, insbesondere Tetraethoxysilan. Darüber hinaus eignen sich aber auch Organotrialkoxysilane; wie sie etwa zur Modifizierung von Kieselgelen bekannt sind. Die Organe-Gruppen in diesen Verbindungen können aliphatische Reste mit 1-20 C-Atomen sein, gegebenenfalls funktionalisiert, etwa durch Hydroxy-, Thio-, Amino-, Carboxyl-Gruppen oder Halogen sowie Alkenylreste. Der Einbau funktionalisierter Organogruppen in die $SiO_2$-Matrix der Teilchen ermöglicht im übrigen eine spätere weitere Modifizierung durch kovalente Bindungsknüpfung in bekannter Weise. Beispiele für derartige Organotrialkoxysilane sind etwa Methyltriethoxysilan, Ethyltriethoxysilan, Hexyltriethoxysilan, Octyltriethoxysilan, Dodecyltriethoxysilan, Octadecyltriethoxysilan, Vinyltriethoxysilan, 3-Hydroxypropyltriethoxysilan, 3-Chloropropyltriethoxysilan, 3-Aminopropyltriethoxysilan, 3-Glycidoxypropyltriethoxysilan, 3-Mercaptopropyltriethoxysilan, 3-Isothiocyanatopropyltriethoxysilan, 3-(Aminoethyl amino)-propyltriethoxysilan, 3-Methacryloxypropyltriethoxysilan, 3-Acetoxypropyltriethoxysilan, N-(3-Triethoxysilylpropyl)-N'-(1-Phenyl-1 -hydroxyisopropyl)-thioharnstoff, N-(3-Triethoxysilylpropyl)-N'-( U-Phenylethy1)-thioharnstoff oder Mischungen hieraus.

In der einfachsten Ausführungsform der erfindungsgemäßen Verfahren werden zunächst die organischen Verbindungen mit der Substanz mit mindestens zwei durch eine Spacer-Gruppe getrennten reaktiven Gruppen umgesetzt und die daraus erhaltene funktionalisierte Verbindung isoliert. Entsprechende Synthesevorschriften finden sich beispielsweise in EP 1 205 177. Im folgenden Schritt wird ein entsprechender Precursor mit der im ersten Schritt erhaltenen Verbindung gemeinsam oder einzeln in ein die Co-Polymeristaion initiierendes Gemisch, vorzugsweise ein Hydrolysegemisch, eingebracht, wobei der Precursor und die Verbindung im Falle der einzelnen Zugabe simultan oder nacheinander zugegeben werden können. Vorzugsweise erfolgt die Zugabe in Form einer Mischung des Precursors und der funktionalisierten Verbindung. Das die Co-Polymeristaion initiierende Gemisch kann bereits alle zum Start der Polymerisation benötigten Komponenten enthalten, es können aber auch einzelne Bestandteile sukzessiv zugegeben werden. Im Falle der bevorzugt eingesetzten hydrolytischen Polykondensation liegt das Hydrolysegemisch vorzugsweise in Form eines Ethanol/Wasser/Ammoniak-Gemisches vor, in das der Precursor und die funktionalisierte Verbindung unter Start der Polykondensation eingebracht werden. Weitere Varianten der Prozessführung unterliegen dem Fachwissen des Fachmanns und können einfach abgeleitet werden. Die Reaktion kann bei Temperaturen im Bereich von 5 bis 80˚C erfolgen, vorzugsweise erfolgt sie im Bereich von 20 bis 80˚C, und insbesondere bevorzugt bei 25 bis 65˚C. Die erhaltenen Partikel können nach dem Fachmann gängigen Methoden isoliert werden, z.B. durch Waschen, Extrahieren, Zentrifugieren. Vorzugsweise werden die erfindungsgemäßen Partikel durch Extrahieren und/oder Zentrifugieren isoliert.

In einem weiteren Schritt wird die Oberfläche der Partikel zusätzlich funktionalisiert. Hierzu werden organische Verbindungen an der Oberfläche der Partikel kovalent gebunden, wobei die organischen Verbindungen vorzugsweise ausgewählt sind aus der Gruppe der lichtabsorbierenden organischen Verbindungen, der Verbindungen mit antioxidanten und/oder radikalinhibierenden Eigenschaften, der Repellentien, der Konservierungsmittel. Beispiele für geeignete Verbindungen dieser Art sind bereits vorab beschrieben.

Die zusätzliche Oberflächenfunktionalisierung erfolgt idealerweise durch Umsetzung der Partikel mit den entsprechenden organischen Verbindungen, vorzugsweise mit den gemäß Verfahrensschritt a) des erfindungsgemäßen Verfahrens erhaltenen funktionalisierten Verbindungen. Die funktionalisierten Verbindungen weisen eine ausreichende Reaktivität aus, um mit der Oberfläche der erfindungsgemäßen Partikel reagieren zu können. Verfahren zur nachträglichen Funktionalisierung sind dem Fachmann bekannt, Beispiele finden sich unter anderem in EP 1 205 177. Im einfachsten Falle, z.B. bei der hydrolytischen Polykondensation, werden die erfindüngsgemäßen Partikel isoliert und unter den bereits beschriebenen Hydrolysebedingungen erneut mit den funktionalisierten Verbindungen umgesetzt. Dieser zusätzliche Verfahrensschritt kann nach der Isolierung der erfindungsgemäßen Partikel erfolgen, alternativ kann die Funktionalisierung auch direkt an die Synthese der erfindungsgemäßen Partikel angeschlossen werden. Diese Vorgehensweise ist bevorzugt. Auf diese Weise können die einzelnen Prozessschritte miteinander kombiniert werden, was ein wesentliche Vereinfachung der Verfahrensführung und eine Einsparung an benötigten Ausgangsstoffen, z.B. in Form des Hydrolysegemisches, ermöglicht. Die zusätzliche Funktionalisierung kann wahlweise auf der Umsetzung der gebildeten Partikel mit noch nicht umgesetzten Anteilen der bei der Herstellung der Partikel eingesetzten Verbindungen beruhen. Alternativ ist es auch möglich, die Funktionalisierung durch zusätzliche Zugabe weiterer Anteile der organischen Verbindungen zu erzielen, insbesondere wenn eine möglichst vollständige Funktionalisierung der Oberfläche der Partikel erwünscht ist.

Die erfindungsgemäßen Partikel eignen sich grundsätzlich zur Verwendung in jeglicher Form von Formulierungen oder Zubereitungen, wie z.B. kosmetischen Zubereitungen aber auch in Zubereitungen, die im technischen Sektor eingesetzt werden können, wie z.B. Farben oder Lacken. Bevorzugt werden die erfindungsgemäßen Partikel in topischen Zubereitungen, insbesondere in der Kosmetik, eingesetzt. Die erfindungsgemäßen Zubereitungen enthalten vorzugsweise neben den Partikeln mindestens einen kosmetisch, pharmazeutisch und/oder dermatologisch verträglichen Träger und/oder Hilfsstoff. Die erfindungsgemäßen Partikel erlauben vorzugsweise die Herstellung von Zubereitungen mit Lichtschutzeigenschaften für eine Vielzahl von Applikationsvarianten und -medien, wobei durch die geeignete Wahl der organischen Verbindung, in diesem Falle der UV-Filter, die Lichtschutzeigenschaften individuell ausgewählt werden können.

Bevorzugte Zubereitungen mit Lichtschutzeigenschaften enthalten dabei zusätzlich mindestens einen weiteren organischen und/oder anorganischen UV-Filter, vorzugsweise ein Dibenzoylmethanderivat. Die im Rahmen der vorliegenden Erfindung verwendeten Dibenzoylmethanderivate sind an sich bereits wohlbekannte Produkte, die insbesondere in den Druckschriften FR-A-2 326 405, FR-A-2 440 933 und EP-A-0 114 607 beschrieben sind.

Die erfindungsgemäß verwendbaren Dibenzoylmethanderivate können insbesondere unter den Dibenzoylmethanderivaten der folgenden Formel ausgewählt sein:

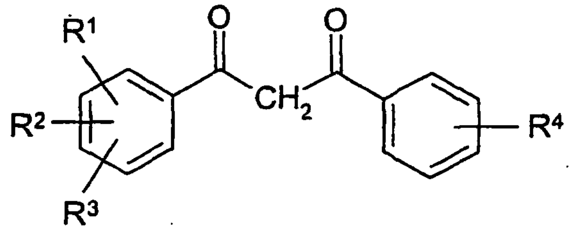

worin $R^1$, $R^2$, $R^3$ und $R^4$, die identisch oder voneinander verschieden sind, Wasserstoff, eine geradkettige oder verzweigte $C_{1-8}$-Alkylgruppe oder eine geradkettige oder verzweigte $C_{1-8}$-Alkoxygruppe bedeuten. Gemäß der vorliegenden Erfindung können selbstverständlich ein Dibenzoylmethanderivat oder mehrere Dibenzoylmethanderivate verwendete werden. Von den Dibenzoylmethanderivaten, auf die sich die vorliegende Erfindung spezieller bezieht, können insbesondere:

- 2-Methyldibenzoylmethan,
- 4-Methyldibenzoylmethan,
- 4-Isopropyldigenzoylmethan,
- 4-tert.-Butyldibenzoylmethan,
- 2;4-Dimethyldibenzoylmethan,
- 2,5-Dimethyldibenzoylmethan,
- 4,4'-Diisopropyldibenzoylmethan,
- 4,4'-Methoxy-tert.-butyldibenzoylmethan,
- 2-Methyl-5-isopropyl-4'-methoxydibenzoylmethan,
- 2-Methyl-5-tert.-butyl-4'-methoxydibenzoylmethan,
- 2,4-Dimethyl-4'-methoxydibenzoylmethan
  und 2,6-Dimethyl-4-tert.-butyl-4'-methoxydibenzoylmethan genannt werden,

wobei diese Aufzählung nicht einschränkend ist. Von den obengenannten Dibenzoylmethanderivaten wird erfindungsgemäß insbesondere das 4,4'-Methoxy-tert.-butyldibenzoylmethan und insbesondere das unter der Handelsbezeichnung Eusolex® 9020 von der Firma Merck KGaA im Handel befindliche 4,4'-Methoxy-tert.-butyldibenzoylmethan bevorzugt, wobei dieses Filter der folgenden Strukturformel entspricht:

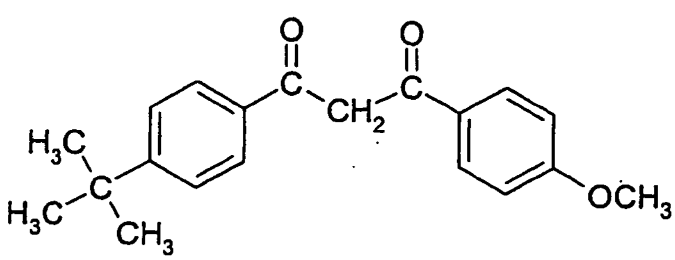

Ein weiteres erfindungsgemäß bevorzugtes Dibenzoylmethanderivat ist das 4-Isopropyldibenzoylmethan.

Weitere bevorzugte Zubereitungen mit Lichtschutzeigenschaften enthalten dabei mindestens ein Benzophenon oder Derivat des Benzophenon, wie insbesondere bevorzugt 2-Hydroxy-4-methoxybenzophenon (z.B. Eusolex® 4360)

oder 2-Hydroxy-4-methoxybenzophenon-5-sulfonsäure und ihr Natriumsalz (z.B. Uvinul® MS-40).

Das oder die Dibenzoylmethanderivat(e) oder das oder die Benzophenonderivat(e) können in den erfindungsgemäßen Zusammensetzungen in Mengenanteilen vorliegen, die im Allgemeinen im Bereich von 0.1 bis 10 Gew.-% liegen und vorzugsweise in Mengenanteilen, die im Bereich von 0.3 bis 5 Gew.-% liegen, wobei diese Mengenanteile auf das Gesamtgewicht der Zusammensetzung bezogen sind.

Weiter kann es erfindungsgemäß bevorzugt sein, wenn die Zubereitungen weitere anorganische UV-Filter enthalten. Hierbei sind sowohl solche aus der Gruppe der Titandioxide. wie z.B. gecoatetes Titandioxid (z.B. Eusolex® T-2000, Eusolex®T-AQUA), Zinkoxide (z.B. Sachtotec®), Eisenoxide oder auch Ceroxide bevorzugt. Diese anorganischen UV-Filter werden in der Regel in einer Menge von 0.5 bis 20 Gewichtsprozent, vorzugsweise 2-10 %, in kosmetische Zubereitungen eingearbeitet. Insbesondere kann es dabei bevorzugt sein, wenn in Emulsionen in eine Phase erfindungsgemäße Partikel und in der anderen Phase ein weiterer anorganischer UV-Filter eingearbeitet wird.

Erfindungsgemäß können die oben genannten UV-Filter auch mit einer die hydrophilen oder die hydrophoben Eigenschaften verstärkenden Oberflächenbehandlung versehen sein. So kann durch die Aufbringung entsprechender Verbindungen eine Hydrophobisierung oder Hydrophilisierung der Partikeloberflächen erzielt werden. Zur hydrophoben Modifikation eignet sich beispielsweise eine Beschichtung mit organischen Säuren, wie z.B. Stearinsäure oder Laurinsäure, mit LCST-Polymeren, organischen Fluoralkoholphosphaten oder eine Silicon- oder Silan-Beschichtung.

Die Silicone sind bekanntlich silizium-organische Polymere oder Oligomere mit geradkettiger oder cyclischer, verzweigter oder vernetzter Struktur mit unterschiedlichen Molekülgewichten, die durch Polymerisation und/oder Polykondensation mit geeignet funktionalisierten Silanen erhalten werden und im wesentlichen aus wiederkehrenden Haupteinheiten gebildet werden, in denen die Siliziumatome über Sauerstoffatome miteinander verknüpft sind (Siloxanbindung), wobei gegebenenfalls substituierte Kohlenwasserstoffgruppen über ein Kohlenstoffatom direkt an die Siliziumatome gebunden sind. Die gebräuchlichsten Kohlenwasserstoffgruppen sind Alkylgruppen und insbesondere Methylgruppen, Fluoralkylgruppen, Acrylgruppen und insbesondere Phenylgruppen sowie Alkenylgruppen und insbesondere Vinylgruppen. Weitere Typen von Gruppen, die entweder direkt oder über eine Kohlenwasserstoffgruppe an die Siloxankette gebunden werden können, sind insbesondere Wasserstoff, die Halogene und insbesondere Chlor, Brom oder Fluor, die Thiole, Alkoxygruppen, Polyoxyalkylengruppen (oder Polyether) und insbesondere Polyoxyethylen und/oder Polyoxypropylen, Hydroxygruppen oder Hydroxyalkylgruppen, gegebenenfalls substituierte Aminogruppen, Amidgruppen, Acyloxygruppen oder Acyloxyalkylgruppen, Hydroxyalkylaminogruppen oder Aminoalkylgruppen, quaternäre Ammoniumgruppen, amphotere Gruppen oder Betaingruppen, anionische Gruppen, wie Carboxylate, Thioglykolate, Sulfosuccinate, Thiosulfate, Phosphate und Sulfate, wobei diese Aufzählung selbstverständlich in keiner Weise einschränkend ist (sogenannte 'organomodifizierte' Silicone).

Im Sinne der vorliegenden Erfindung sollen mit dem Ausdruck 'Silicone' auch die zu ihrer Herstellung benötigten Silane und insbesondere die Alkylsilane eingeschlossen und abgedeckt sein.

Die für die vorliegende Erfindung geeigneten Silicone, die zum Umhüllen der UV-Schutzmittel verwendet werden können, sind vorzugsweise unter den Alkylsilanen, den Polydialkylsiloxanen und den Polyalkylhydrogensiloxanen ausgewählt. Noch bevorzugter sind die Silicone unter Octyltrimethylsilan, den Polydimethylsiloxanen und den Polymethylhydrogenosiloxanen ausgewählt.

Die UV-Schutzmittel können in den erfindungsgemäßen Zusammensetzungen in Mengenanteilen vorliegen, die im allgemeinen im Bereich von 0.1 bis 50 Gew.-% liegen und vorzugsweise in Mengenanteilen, die im Bereich von 0.5 bis 20 Gew.-% liegen, wobei diese Mengenanteile auf das Gesamtgewicht der Zusammensetzung bezogen sind.

In einer weiteren ebenfalls bevorzugten Ausführungsform der vorliegenden Erfindung enthält die erfindungsgemäße Zubereitung mindestens einen Setbstbräuner.

Als vorteilhafte Selbstbräuner können unter anderem eingesetzt werden:

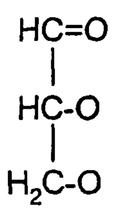

Glycerolaldehyd

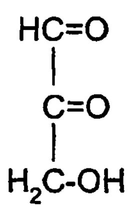

Hydroxymethylglyoxal

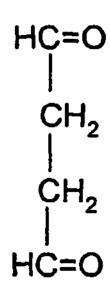

γ-Dialdehyd

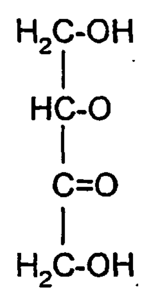

Erythrulose

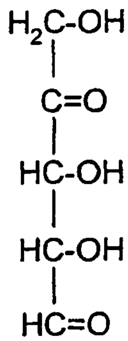

6-Aldo-D-Fructose

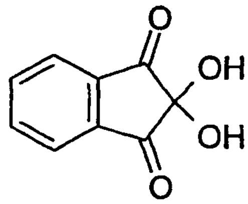

Ninhydrin

Ferner ist das 5-Hydroxy-1,4-naphtochinon (Juglon) zu nennen, das aus den Schalen frischer Walnüsse extrahiert wird

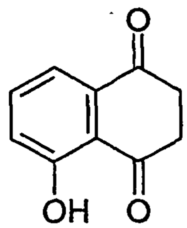

5-Hydroxy-1,4-naphtochinon (Juglon)

sowie das in den Henna-Blättern vorkommende 2-Hydroxy-1,4-naphtochinon (Lawson).

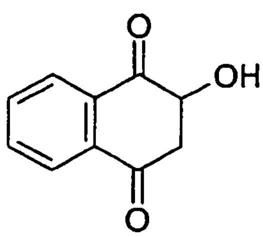

2-Hydroxy-1,4-naphtochinon (Lawson)

Ganz besonders bevorzugt ist das 1,3-Dihydroxyaceton (DHA), ein im menschlichen Körper vorkommender dreiwertiger Zucker und dessen Derivate.

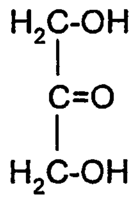

1,3-Dihydroxyaceton (DHA)

Die Verwendung von erfindungsgemäßen Partikeln bei der Stabilisierung von Selbstbräunern, insbesondere Dihydroxyaceton oder Dihydroxyacetonderivaten ist ein weiterer Gegenstand der vorliegenden Erfindung.

Ferner können die erfindungsgemäßen Zubereitungen auch Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z.B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| **Chemische oder sonstige Bezeichnung** | **CIN** | **Farbe** |
|---|---|---|
| Pigment Green | 10006 | grün |
| Acid Green 1 | 10020 | Grün |
| 2,4-Dinitrohydroxynaphthalin-7-sulfonsäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'sulfonsäurediethylamid-1'-phenylazo)-3-hydroxy-5"-chloro-2",4"-dimethoxy2-naphthoesäureanilid | 12490 | Rot |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure)-1-hydroxynaphthalin-4-sulfosäure | 14700 | Rot |
| 2-(4-Sulfo-1-naphthylazo)-1-naphthol-4-sutfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäu re-4-chlor-5-carbonsäure-1-phenylazo)-2- hydroxynaphthalin | 15525 | Rot |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | rot |

(fortgesetzt)

| Chemische oder sonstige Bezeichnung | CIN | Farbe |
|---|---|---|
| 1-(2-Sulfo-4-methyl-5-chlor-1-phenylazo)-2-hydroxy-naphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2-phenylazo-1-naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxy-pyrazolon-3-carbonsäure | 19140 | Gelb |
| Pigment Yellow 16 | 20040 | Gelb |
| 2,6-(4'-Sulfo-2",4"-dimethyl)-bis-phenylazo)1,3-dihydroxybenzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1 "-phenylazo)-7'-sulfo-1'-naphthylazo]-1-hydroxy-7-aminonaphthalin-3,6-disulfosäure | 27755 | schwarz |
| 4-[4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-ß-Apo-8'-Carotinaldehyd ($C_{30}$) | 40820 | Orange |
| trans-Apo-8'-Carotinsäure ($C_{30}$)-ethylester | 40850 | Orange |
| Canthaxanthin | 40850 | Orange |
| Acid Blue 1 | 42045 | Blau |
| 2,4-Disulfo-5-hydroxy-4'-4"-bis-(diethylamino)triphenyl-carbinol | 42051 | Blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl) $\Delta^{2,5}$-cyclohexadienimin | 42090 | Blau |
| Acid Green 9 | 42100 | Grün |
| Diethyl-di-sulfobenzyl-di-4-amino-2-chlor-di-2-methyl-fuchsonimrnonium | 42170 | Grün |
| Basic Violet 14 | 42510 | Violet |
| Basic Violet 2 | 42520 | Violet |
| 2'-Methyl-4'-(N-ethyl-N-m-sulfobenzyl)-amino-4"-(N-diethyl)- amino-2-methyl-N-ethyllN-m-sulfobenzyl-fuchsonimmonium | 42735 | Blau |
| 4'-(N-Dimethyl)-amino-4"-(N-phenyl)-aminonaphtho-N-dimethylfuchsonimmonium | 44045 | Blau |
| 2-Hydroxy-3,6-disulfo-4,4'-bis-dimethylaminonaphthofuchsonimmonium | 44090 | Grün |

(fortgesetzt)

| Chemische oder sonstige Bezeichnung | CIN | Farbe |
|---|---|---|
| Acid Red 52 | 45100 | Rot |
| 3-(2'-Methylphenylamino)-6-(2'-methyl-4'-sulfophenylamino)-9-(2"-carboxyphenyl)-xantheniumsalz | 45190 | Violet |
| Acid Red 50 | 45220 | Rot |
| Phenyl-2-oxyfluoron-2-carbonsäure | 45350 | gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |
| Acid Violet 50 | 50325 | Violett |
| Acid Black 2 | 50420 | Schwarz |
| Pigment Violet 23 | 51319 | Violett |
| 1,2-Dioxyanthrachinon, Calcium-Sluminiumkomplex | 58000 | Rot |
| 3-Oxypyren-5,8,10-sulfosäure | 59040 | Grün |
| 1-Hydroxy-4-N-phenyl-aminoanthrachinon- | 60724 | Violett |
| 1-Hydroxy-4-(4'-methylphenylamino)-anthrachinon | 60725 | Violett |
| Acid Violet 23 | 60730 | Violett |
| 1,4-Di(4'-methyl-phenylamino)-anthrachinon | 61565 | Grün |
| 1,4-Bis-(o-sulfo-p-toluidino)-anthrachinon | 61570 | Grün |
| Acid Blue 80 | 61585 | Blau |
| Acid Blue 62 | 62045 | Blau |
| N,N'-Dihydro-1,2,1',2'-anthrachinonazin | 69800 | Blau |
| Vat Blue 6; Pigment Blue 64 | 69825 | Blau |
| Vat Orange 7 | 71105 | orange |
| Indigo | 73000 | Blau |
| Indigo-disulfosäure | 73015 | Blau |
| 4,4'-Dimethyl-6,6'-dichlorthioindigo | 73360 | Rot |
| 5,5'Dichlor-7,7'-dimethylthioindigo | 73385 | violett |
| Quinacridone Violet 19 | 73900 | violett |
| Pigment Red 122 | 73915 | Rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6, 19; Natural Red 1 | 75100 | gelb |
| Bixin, Nor-Bixin | 75120 | orange |
| Lycopin | 75125 | gelb |
| trans-alpha-, bet- bzw. gamma-Carotin - | 75130 | orange |
| Keto- und/oder Hydroxylderivate des Carotins . | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | 1 Rot |
| Chlorophyll a und b; Kupferverbindungen der Chlorophylle und Chlorophylline | 75810 | grün |

(fortgesetzt)

| Chemische oder sonstige Bezeichnung | CIN | Farbe |
|---|---|---|
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | Rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 :1 | schwarz |
| Chromoxid | 77288 | grün |
| Chromoxid, wasserhaltig | 77278 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyahoferrat | 77510 | blau |
| Pigment White 18 | 77713 | weiß |
| Mangananimoniumdiphosphat | 77742 | violett |
| Manganphosphat; $Mn_3(PO_4)_2$. 7 $H_2O$ | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalzem, Anthocyane | | rot |
| Aluminium-, Zink-, Magnesium- und Calciumstearat | | weiß |
| Bromthymolblau | | blau |

Es kann ferner günstig sein, als Farbstoff eine oder mehrerer Substanzen aus der folgenden Gruppe zu wählen: 2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfonsäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfonsäure, Aluminiumsalz der 4-(4-Sulfo-1-phenylazo)-2-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfonsäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z.B. Paprikaextrakt, β-Carotin oder Cochenille.

Vorteilhaft im Sinne der vorliegenden Erfindung sind ferner Gelcrémes mit einem Gehalt an Effektpigmenten.

Bevorzugt sind insbesondere die im folgenden aufgelisteten Arten von Effektpigmenten:

1. Natürliche Effektpigmente, wie z.B.

   a) "Fischsilber" (Guanin/Hypoxanthin-Mischkristalle aus Fischschuppen) und
   b) "Perlmutt" (vermahlene Muschelschalen)

2. Monokristalline Effektpigmente, wie z.B. Bismutoxychlorid (BiOCl)
3. Schicht-Substrat Pigmente: z.B. Glimmer/Metalloxid

Basis für Effektpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z.B. das unter der CIN 77163 aufgelistete Glanzpigment.

Vorteilhaft sind ferner beispielsweise die folgenden Effektpigmentarten auf Basis von Glimmer/Metalloxid:

| Gruppe | **Belegung/Schichtdicke,** | **Farbe** |
|---|---|---|
| **Silberweiße Effektpigmente** | $TiO_2$: 40-60 nm | silber |
| **Interferenzpigmente** | $TiO_2$: 60-80 nm | gelb |
| | $TiO_2$ 80-100 nm | rot |
| | $TiO_2$:100-140 nm | blau |
| | $TiO_2$: 120-160 nm | grün |
| **Farbglanzpigmente** | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| **Kombinationspigmente** | $TiO_2$ / $Fe_2O_3$ | Goldtöne |
| | $TiO_2$/ $Cr_2O_3$ | grün |
| | $TiO_2$/ Berliner Blau | tiefblau |

Besonders bevorzugt sind z.B. die von der Firma Merck KGaA unter den Handelsnamen Timiron®, Colorone® oder Dichrona® erhältlichen Perlglanzpigmente.

Die Liste der genannten Effektpigmente soll selbstverständlich nicht limitierend sein. Im Sinne der vorliegenden Erfindung vorteilhafte Effektpigmente sind auf zahlreichen, an sich bekannten Wegen erhältlich. Darüber hinaus lassen sich beispielsweise auch andere Substrate außer Glimmer mit weiteren Metalloxiden beschichten, wie z.B. Silica und dergleichen mehr. Vorteilhaft sind z.B. mit $TiO_2$ und $Fe_2O_3$ beschichtete $SiO_2$-Partikel ("Ronaspheren"), die von der Firma Merck KGaA vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Effektpigmente, welche unter der Verwendung von $SiO_2$ oder $Al_2O_3$ hergestellt werden. Solche Pigmente, die auch zusätzlich goniochromatische Effekte haben können, sind z.B. unter dem Handelsnamen Colorstream® oder Xirallic® bei der Firma Merck KGaA erhältlich.

Weiterhin vorteilhaft können Pigmente der Firma Engelhard auf Basis von Calcium Natrium Borosilikat, die mit Titandioxid beschichtet sind, eingesetzt werden. Diese sind unter dem Namen Reflecks® erhältlich. Sie weisen durch ihre Partikelgröße von 40-80 $\mu$m zusätzlich zu der Farbe einen Glitzereffekt auf.

Besonders vorteilhaft sind ferner auch Effektpigmente, welche unter der Handelsbezeichnung Metasomes® Standard/Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte

hervorgerufen werden. Die Gesamtmenge der Farbstoffe und farbgebenden Pigmente wird vorteilhaft aus dem Bereich von z.B. 0.1 Gew.-% bis 30 Gew.-%, vorzugsweise von 0.5 bis 15 Gew.-%, insbesondere von 1.0 bis 10 Gew.-% gewählt, jeweils bezogen auf das Gesamtgewicht der Zubereitungen.

Die erfindungsgemäßen Zubereitungen können selbstverständlich einen oder mehrere zusätzliche(n) hydrophile(n) oder lipophile(n) Sonnenschutzfilter, die im UV-A-Bereich und/oder UV-B-Bereich und/oder IR und/oder VIS-Bereich (Absorber) wirksam sind, enthalten. Diese zusätzlichen Filter können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben.

Prinzipiell kommen alle UV-Filter für eine Kombination mit den erfindungsgemäßen Partikeln in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B. Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxobom-3-ylidenmethyl)anilinium methylsulfat (z.B. Mexoryl® SK) oder (2-Oxoborn-3-yliden)toluol-4-sulfonsäure (z.B. Mexoryl® SL), Methoxyzimtsäureester wie Methoxyzimtsäureoctylester (z.B. Eusole)® 2292), 4-Methoxyzimtsäureisopentylester, z.B. als Gemisch der Isomere (z.B. Neo Heliopan® E 1000), Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS), 4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25), Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure, und weitere Substanzen wie

- 2-Cyano-3,3-diphenylacrylsäure-2-ethylhexylester (z.B. Eusolex® OCR),
- 3,3'-(1,4-Phenylendimethylen)-bis-7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin ( z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden. Insbesondere können vorteilhaft auch organische partikuläre UV-Filter, wie Sie beispielsweise in der Patentanmeldung WO 99/66896 beschrieben sind, mit den erfindungsgemäßen Pudern kombiniert werden.

Diese organischen UV-Filter werden in der Regel in einer Menge von 0.5 bis 20 Gewichtsprozent, vorzugsweise 1-10 Gew.-%, in kosmetische Formulierungen eingearbeitet.

Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- α-(Trimethylsilyl)-ω-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1 )
- 2,2'-Methylen-bis-(6-(2H-benzotriazol-2-yl)-4-(1,1,3,3-tetramethyl-butyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-meth-oxy-benzophenon, Methoxyzimtsäureoctylester, 3,3,5-Trimethyl-cyclo-hexylsali-cylat, 4-(Dimethylamino)benzoesäure=2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanolaminsalze.

Bevorzugte Zubereitungen können auch Verbindungen der Formel I enthalten,

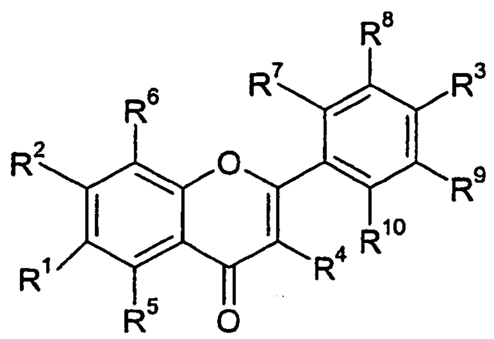

I

wobei $R^1$ und $R^2$ ausgewählt sind aus
- H

- und $OR^{11}$, wobei $OR^{11}$ unabhängig voneinander steht für
  - OH
  - geradkettige oder verzweigte $C_1$- bis $C_{20}$-Alkyloxygruppen,
  - geradkettigen oder verzweigten $C_3$- bis $C_{20}$-Alkenyloxygruppen,
  - geradkettigen oder verzweigten $C_1$- bis $C_{20}$-Hydroxyalkoxygruppen, wobei die Hydroxygruppe(n) an primäre oder sekundäre Kohlenstoffatome der Kette gebunden sein können und weiter die Alkylkette auch durch Sauerstoff unterbrochen sein kann, und/oder
  - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
- Mono- und/oder Oligoglycosylreste,
  mit der Maßgabe, dass mindestens ein Rest aus $R^1$ und $R^2$ steht für $OR^{11}$,
  und $R^3$ steht für einen Rest $OR^{11}$ und
  $R^4$ bis $R^7$ und $R^{10}$ gleich oder verschieden sein können, und unabhängig voneinander stehen für
  - H
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen,

wobei die Hydroxygruppe an ein primäres oder sekundäres Kohlenstoffatom der Kette gebunden sein kann und weiter die Alkylkette auch durch Sauerstoff unterbrochen sein kann, und/oder

- $C_3$- bis $C_{10}$-Cycloalkylgruppen und/oder $C_3$- bis $C_{12}$-Cycloalkenyl-gruppen, wobei die Ringe jeweils auch durch $-(CH_2)_n$-Gruppen mit n = 1 bis 3 überbrückt sein können und
  $R^8$ und $R^9$ gleich oder verschieden sein können, und unabhängig voneinander stehen für
  - H
  - $OR^{11}$
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen,

wobei die Hydroxygruppe an ein primäres oder sekundäres Kohlenstoffatom der Kette gebunden sein kann und weiter die Alkylkette auch durch Sauerstoff unterbrochen sein kann, und/oder

- $C_3$- bis $C_{10}$-Cycloalkylgruppen und/oder $C_3$- bis $C_{12}$-Cycloalkenyl-gruppen, wobei die Ringe jeweils auch durch $-(CH_2)_n$-Gruppen mit n = 1 bis 3 überbrückt sein können.

Vorteile der erfindungsgemäßen Zusammensetzungen sind dabei insbesondere die UV-Licht filternde Wirkung und die gute Hautverträglichkeit. Zusätzlich sind die hier beschriebenen Verbindungen der Formel I farblos oder nur schwach gefärbt und führen so, im Gegensatz zu vielen bekannten natürlich vorkommenden Flavonoiden, nicht zu Verfärbungen der Zubereitungen.

Unter den erfindungsgemäß einzusetzenden Flavonoiden der Formel I finden sich dabei Breitband-UV-Filter, andere ebenfalls bevorzugte Verbindungen der Formel I zeigen ein Absorptionsmaximum im Grenzbereich zwischen

der UV-B- und der UV-A-Strahlung. Als UV-A-II-Filter ergänzen sie daher vorteilhaft das Absorptionsspektrum von handelsüblichen UV-B- bzw. UV-A-I-Filtern. Bevorzugte erfindungsgemäße Zubereitungen mit Lichtschutzeigenschaften enthalten zumindest eine Verbindung der Formel I, wobei $R^3$ steht für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste; vorzugsweise Glucosylreste und

$R^1$ und/oder $R^2$ vorzugsweise stehen für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste.

Diese bevorzugten Verbindungen zeichnen sich durch eine besonders intensive UV-Absorption aus.

Zusätzlich haben solche bevorzugten Verbindungen Vorteile bei der Einarbeitung in die Zubereitungen:

- Mono- und/oder Oligoglycosylreste verbessern die Wasserlöslichkeit der erfindungsgemäß einzusetzenden Verbindungen; geradkettige oder verzweigte $C_1$- bis $C_{20}$-Alkoxygruppen, insbesondere die langkettigen Alkoxyfunktionen, wie Ethylhexyloxy-Gruppen erhöhen die Öllöslichkeit der Verbindungen;
  d.h. über die geeignete Auswahl der Substituenten kann die Hydrophilie bzw. Lipophilie der Verbindungen nach Formel I gesteuert werden. Als Mono- oder Oligosaccharidreste bevorzugt sind dabei Hexosylreste, insbesondere Ramnosylreste und Glucosylreste. Aber auch andere Hexosylreste, beispielsweise Allosyl, Altrosyl, Galactosyl, Gulosyl, Idosyl, - Mannosyl und Talosyl sind gegebenenfalls vorteilhaft zu verwenden. Es kann auch vorteilhaft sein, Pentosylreste zu verwenden. Die Glycosylreste können α- oder β-glycosidisch mit dem Grundkörper verbunden sein. Ein bevorzugtes Disaccharid ist beispielsweise das 6-O-(6-deoxy-α-L-mannopyranosyl)-β-D-glucopyranosid.

Es hat sich gezeigt, dass die Intensität der UV-Absorption insbesondere dann hoch ist, wenn $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$-bis $C_{20}$-Alkoxygruppen, vorzugsweise Methoxy, Ethoxy oder Ethylhexyloxy. Daher sind Zubereitungen mit Lichtschutzeigenschaften enthaltend zumindest eine Verbindung der Formel I, die dadurch gekennzeichnet ist, dass $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$- bis $C_{20}$-Alkoxygruppen, vorzugsweise Methoxy, Ethoxy oder Ethylhexyloxy, erfindungsgemäß besonders bevorzugt. Insbesondere ist es dabei bevorzugt, wenn und $R^8$ und $R^9$ für H stehen.

Die Verbindungen der Formel I werden erfindungsgemäß typisch in Mengen von 0.01 bis 20 Gew.-%, vorzugsweise in Mengen von 0.5 Gew.-% bis 10 Gew.-% und insbesondere bevorzugt in Mengen von 1 bis 8 Gew.-% eingesetzt. Dabei bereitet es dem Fachmann keinerlei Schwierigkeiten, die Mengen abhängig von dem beabsichtigten Lichtschutzfaktor der Zubereitung entsprechend auszuwählen.

Durch Kombination von Partikeln mit weiteren UV-Filtem kann die Schutzwirkung gegen schädliche Einwirkungen der UV-Strahlung optimiert werden.

Alle genannten UV-Filter einschließlich der Verbindungen der Formel I können ebenfalls auch in verkapselter Form eingesetzt werden. Insbesondere ist es von Vorteil organische UV-Filter in verkapselter Form einzusetzen. Im Einzelnen ergeben sich durch die Verkapselung die folgenden Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.

- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.

- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.

- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher kann es erfindungsgemäß bevorzugt sein, wenn einer oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen Sol-Gel-Prozeß, wie er in den Anmeldungen EP 1 382 328, WO 00/09652, WO 00/72806 und WO 00/71084 beschrieben ist, erhalten werden können. Bevorzugt sind hier wiederum Kapseln, deren Wände aus Kieselgel (Silica; undefiniertes Siliziumoxidhydroxid) oder Siliciumdioxid aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört. Darüber hinaus können auch diese Kapseln nachbehandelt sein, das heißt die Oberfläche der Partikel ist hydrophobisiert oder hydrophilisiert. Beispiele für derartige Nachbehandlungen sind bereits genannt.

Weisen die erfindungsgemäßen Zubereitungen Verbindungen entsprechend Formel I mit freien Hydroxy-Gruppen auf, so zeigen sie neben den beschriebenen Eigenschaften zusätzlich eine Wirkung als Antioxidans und/oder Radikalfänger. Bevorzugt sind daher auch Zubereitungen mit Lichtschutzeigenschaften enthaltend zumindest eine Verbindung der Formel I, die dadurch gekennzeichnet ist, dass mindestens einer der Reste $R^1$ bis $R^3$ steht für OH, wobei vorzugsweise mindestens einer der Reste $R^1$ oder $R^2$ für OH steht.

Damit die Verbindungen der Formel I ihre positive Wirkung als Radikalfänger auf die Haut besonders gut entwickeln können, kann es bevorzugt sein die Verbindungen der Formel I in tiefere Hautschichten eindringen zu lassen. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I senkbar. Die Zubereitung wird dann beispielsweise so gestaltet, dass sie für eine orale Gabe geeignet ist.

Allgemein wirken die Substanzen der Formel I als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalarionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Es wird vermutet, dass bevorzugte Verbindungen der Formel I auch als Enzymhemmer wirken. Sie hemmen vermutlich Histidindecarboxylase, Proteinkinasen, Elastase, Aldosereduktase sowie Hyaluronidase, und ermöglichen daher, die Unversehrtheit der Grundsubstanz vaskulärer Hüllen aufrecht zu erhalten. Ferner hemmen sie vermutlich nicht spezifisch Katechol-O-methyltransferase, wodurch die Menge der verfügbaren Katecholamine und dadurch die Gefäßfestigkeit erhöht wird. Weiter hemmen sie AMP-Phosphodiesterase, wodurch die Substanzen ein Potential zur Hemmung der Thrombozytenaggregation aufweisen. Aufgrund dieser Eigenschaften eignen sich die erfindungsgemäßen Zubereitungen allgemein zur Immunprotektion und zum Schutz der DNA und RNA. Insbesondere eignen sich die Zubereitungen dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Zubereitungen ist der Zellschutz, insbesondere der Schutz von Langerhans-Zellen vor Schäden durch die oben genannten Einflüsse. Alle diese Verwendungen bzw. die Verwendung der Verbindungen der Formel I zur Herstellung entsprechend einsetzbarer Zubereitungen sind ausdrücklich auch Gegenstand der vorliegenden Erfindung.

Insbesondere eignen sich bevorzugte erfindungsgemäße Zusammensetzungen auch zur Behandlung von Hautkrankheiten, die mit einer Störung der Keratinisierung verbunden sind, die die Differenzierung und Zellproliferation betrifft, insbesondere zur Behandlung der Akne vulgaris, Akne comedonică, der polymorphen Akne, der Akne rosaceae, der nodulären Akne, der Akne conglobata, der alters-bedingten Aknen, der als Nebenwirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosiformen Zustände, der Darrier-Krankheit, der Keratosis palmoplantaris, der Leukoplasien, der leukoplasiformen Zustände, der Haut- und Schleimhautflechten (Buccal) (Lichen),

zur Behandlung anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger und Zehennägel betreffen, und des psoriatischen Rheumas und der Hautatopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillomatosis florida, und der Wucherungen, die durch UV-Strahlung hervorgerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkrankungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der licht-bedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immunologischen Komponente, zur Behandlung von Hertz-/Kreislauf-Erkran-kungen, wie Arteriosklerose oder Bluthochdruck, sowie des Insulinunabhängigen Diabetes, zur Behandlung von Hautproblemen, die durch UV-Strahlung hervorgerufen werden.

Die schützende Wirkung erfindungsgemäßer Zubereitungen gegen oxidativen Stress bzw. gegen die Einwirkung von Radikalen kann also weiter verbessert werden, wenn die Zubereitungen ein oder mehrere Antioxidantien enthalten, wobei es dem Fachmann keinerlei Schwierigkeiten bereitet, geeignete schnell oder zeitverzögert wirkende Antioxidantien auszuwählen.

In einer bevorzugten Ausführungsform der vorliegenden Erfindungen handelt es sich bei der Zubereitung daher um eine Zubereitung zum Schutz von Körperzellen gegen oxidativen Stress, insbesondere zur Verringerung der Hautalterung, dadurch gekennzeichnet, dass sie neben den einer oder mehreren Verbindungen nach Formel I sein oder mehrere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit Verbindungen der

Formel I in solchen Zusammensetzungen üblicherweise in Verhältnissen im Bereich von 1000:1 bis 1:1000, bevorzugt in Mengen von 100:1 bis 1:100 eingesetzt.

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$). Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C und dessen Derivaten, DL-α-Tocopherol, Tocopherol-E-Acetat, Nicotinsäure, Pantothensäure und Biotin. Vitamine werden dabei mit Verbindungen der Formel I üblicherweise in Verhältnissen im Bereich von 1000:1 bis 1:1000, bevorzugt in Mengen von 100: 1 bis 1:100 eingesetzt.

Unter den Phenolen mit antioxidativer Wirkung sind die teilweise als Naturstoffe vorkommenden Polyphenole für Anwendungen im pharmazeutischen, kosmetischen oder Emährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'-oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C:M. Rietjens; Free Radical Biology & Medicine 2001, 31(7); 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete Antioxidantien sind weiter Verbindungen der Formel II

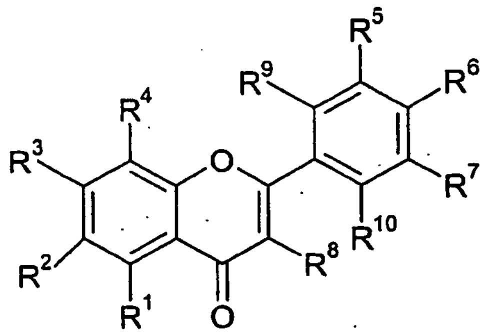

II

wobei $R^1$ bis $R^{10}$ gleich oder verschieden sein können und ausgewählt sind aus

- H
- $OR^{11}$
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,
- wobei alle $OR^{11}$ unabhängig voneinander stehen für
- OH
- geradkettige oder verzweigte $C_1$- bis $C_{20}$-Alkyloxygruppen,
- geradkettigen oder verzweigten $C_3$- bis $C_{20}$-Alkenyloxygruppen,
- geradkettigen oder verzweigten $C_1$- bis $C_{20}$-Hydroxyalkoxygruppen,

wobei die Hydroxygruppe(n) an primäre oder sekundäre Kohlenstoffatome der Kette gebunden sein können und weiter die Alkylkette auch durch Sauerstoff unterbrochen sein kann, und/oder

- $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
- Mono- und/oder Oligoglycosylreste,

mit der Maßgabe, dass mindestens 4 Reste aus $R^1$ bis $R^7$ stehen für OH und dass im Molekül mindestens 2 Paare benachbarter Gruppen -OH vorliegen,

- oder $R^2$, $R^5$ und $R^6$ für OH und die Reste $R^1$, $R^3$, $R^4$ und $R^{7-10}$ für H stehen,

wie sie in der älteren Deutschen Patentanmeldung DE 10244282.7 beschrieben sind.

Vorteile der erfindungsgemäßen Zusammensetzungen enthaltend mindestens ein Antioxidans sind dabei neben den oben genannten Vorteilen insbesondere die antioxidante Wirkung und die gute Hautverträglichkeit. Zusätzlich sind bevorzugte der hier beschriebenen Verbindungen farblos oder nur schwach gefärbt und führen so nicht oder nur in geringer Weise zu Verfärbungen der Zubereitungen. Von Vorteil ist insbesondere das besondere Wirkprofil der Verbindungen nach Formel II, welches sich im DPPH-Assay in einer hohen Kapazität Radikale zu fangen ($EC_{50}$), einer zeitverzögerten Wirkung ($T_{EC50}$ > 120 min) und damit einer mittleren bis hohen antiradikalischen Effizienz (AE) äußert. Zudem vereinigen die Verbindungen nach Formel II im Molekül antioxidative Eigenschaften mit UV-Absorption im UV-A- und/oder -B-Bereich. Bevorzugt sind daher auch Zubereitungen enthaltend zumindest eine Verbindung der Formel II, die dadurch gekennzeichnet ist, dass mindestens zwei benachbarte Reste der Reste $R^1$ bis $R^4$ stehen für OH und mindestens zwei benachbarte Reste der Reste $R^5$ bis $R^7$ stehen für OH. Insbesondere bevorzugte Zubereitungen enthalten zumindest eine Verbindung der Formel II, die dadurch gekennzeichnet ist, dass mindestens drei benachbarte Reste der Reste $R^1$ bis $R^4$ stehen für OH, wobei vorzugsweise die Reste $R^1$ bis $R^3$ für OH stehen.

Als Flavon-Derivate werden erfindungsgemäß Flavonoide und Coumaranone verstanden. Als Flavonoide werden erfindungsgemäß die Glykoside von Flavanonen, Flavonen, 3-Hydroxyflavonen (= Flavonolen), Auronen, Isoflavonen und Rotenoiden aufgefaßt [Römpp Chemie Lexikon, Band 9, 1993]. Im Rahmen der vorliegenden Erfindung werden hierunter jedoch auch die Aglykone, d.h. die zuckerfreien Bestandteile, und die Derivate der Flavonoide und der Aglykone verstanden. Weiterhin wird im Rahmen der vorliegenden Erfindung unter dem Begriff Flavonoid auch Anthocyanidin (Cyanidin) verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und Isoftavonen, insbesondere von Flavanonen, Flavonen, 3-Hydroxyflavonen und Auronen, ab.

Die Flavonoide sind vorzugsweise ausgewählt aus folgenden Verbindungen: 4,6,3',4'-Tetrahydroxyauron; Quercetin, Rutin, Isoquercetin, Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin), α-Glycosylrutin, Tilirosid sowie deren Sulfaten und Phosphaten. Unter den Flavonoiden sind als erfindungsgemäße Aktivstoffe insbesondere Rutin, Tilirosid, α-Glycosylrutin und Troxerutin bevorzugt.

Unter den Coumaranonen ist 4,6,3',4'-Tetrahydroxybenzylcoumaranon-3 bevorzugt.

Unter Chromon-Derivaten werden vorzugsweise bestimmte Chromen-2-on-Derivate, die sich als Wirkstoffe zur vorbeugenden Behandlung von menschlicher Haut und menschlicher Haare gegen Alterungsprozesse und schädigende Umwelteinflüssen eignen, verstanden. Sie zeigen gleichzeitig ein niedriges Irritationspotential für die Haut, beeinflussen die Wasserbindung in der Haut positiv, erhalten oder erhöhen die Elastizität der Haut und fördern somit eine Glättung der Haut. Diese Verbindungen entsprechen vorzugsweise der Formel III

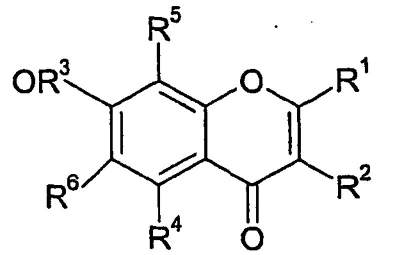

III

wobei

$R^1$ und $R^2$ gleich oder verschieden sein können und ausgewählt sind aus

- H, -C(=O)-$R^7$, -C(=O)-O$R^7$,

- geradkettigen oder verzweigten $C_1$- bis $C_{20}$-Alkylgruppen,
- geradkettigen oder verzweigten $C_3$- bis $C_{20}$-Alkenylgruppen, geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können, $R^3$ steht für H oder geradkettige oder verzweigte $C_1$- bis $C_{20}$-Alkylgruppen, $R^4$ steht für H oder $OR^8$,

$R^5$ und $R^6$ gleich oder verschieden sein können und ausgewählt sind aus

- -H, -OH,
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und

$R^7$ steht für H, geradkettige oder verzweigte $C_1$- bis $C_{20}$-Alkylgruppen, eine Polyhydroxy-Verbindung, wie vorzugsweise einen Ascorbinsäurerest oder glycosidische Reste und

$R^8$ steht für H oder geradkettige oder verzweigte $C_1$- bis $C_{20}$-Alkylgruppen, wobei mindestens 2 der Substituenten $R^1$, $R^2$, $R^4$-$R^6$ verschieden von H sind oder mindestens ein Substituent aus $R^1$ und $R^2$ für $-C(=O)-R^7$ oder$-C(=O)-OR^7$ steht.

Der Anteil an einer oder mehreren Verbindungen ausgewählt aus Flavonoiden, Cromon-Derivaten und Coumaranonen in der erfindungsgemäßen Zubereitung beträgt vorzugsweise von 0.001 bis 5 Gew.-%, besonders bevorzugt von 0.01 bis 2 Gew.-%, bezogen auf die gesamte Zubereitung.

Die erfindungsgemäßen Zubereitungen mit Lichtschutzeigenschaften können darüber hinaus weitere übliche_ hautschonende oder hautpflegende Wirkstoffe enthalten. Dies können prinzipiell alle dem Fachmann bekannten Wirkstoffe sein.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle (E. A. Galinski et al., Eur. J. Biochem., 149 (1985) Seite 135-139*)*. Dabei sind unter den Pyrimidincarbonsäuren, insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösemitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel IV eingesetzt,

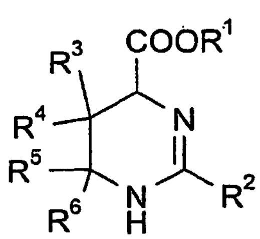

IV

worin $R^1$ ein Rest H oder $C_{1-8}$-Alkyl, $R^2$ ein Rest H oder $C_{1-4}$-Alkyl und $R^3$, $R^4$, $R^5$ sowie $R^6$ jeweils unabhängig voneinander ein Rest aus der Gruppe H, OH, $NH_2$ und $C_{1-4}$-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die Aryloxime, vorzugsweise 2-Hydroxy-5-methyllaurophenonoxim enthalten, zeigen überraschende antiinflammatorische Eignung. Dabei enthalten die Zubereitungen vorzugsweise 0.01 bis 10 Gew.-% des Aryloxims, wobei es insbesondere bevorzugt ist, wenn die Zubereitung 0.05 bis 5 Gew.-% Aryloxim enthält.

Alle hier beschriebenen Verbindungen oder Komponenten, die in den Zubereitungen verwendet werden können, sind entweder bekannt und käuflich erwerbbar oder können nach bekannten Verfahren synthetisiert werden.

Neben den hier beschriebenen Verbindungen können die erfindungsgemäßen Zubereitungen auch mindestens einen Photostabilisator, vorzugsweise entsprechend der Formel V

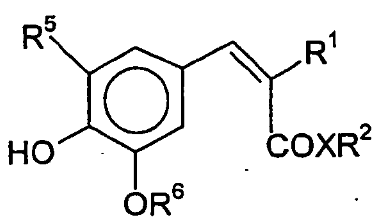

V,

wobei

$R^1$ ausgewählt ist aus $-C(O)CH_3$, $-CO_2R^3$, $-C(O)NH_2$ and $-C(O)N(R^4)_2$; X is O or NH;

$R^2$ steht für einen linearen oder verzweigten $C_{1-30}$-Alkylrest;

$R^3$ steht für einen linearen oder verzweigten $C_{1-20}$-Alkylrest,

alle $R^4$ unabhängig voneinander stehen für H oder lineare oder verzweigte $C_{1-8}$-Alkylreste

$R^5$ steht für H, einen linearen oder verzweigten $C_{1-8}$-Alkylrest oder

einen linearen oder verzweigten $-O-C_{1-8}$-Alkylrest und $R^6$ steht für einen $C_{1-8}$-Alkylrest,

wobei es sich bei dem Photostabilisator insbesondere bevorzugt um 2-(4-Hydroxy-3,5-dimethoxy-benzyliden)-malonsäure-bis-(2-ethyl-hexyl)ester handelt, enthalten. Entsprechende Photostabilisatoren, ihre Herstellung und Verwendung sind in der Internationalen Patentanmeldung WO 03/007906 beschrieben.

Die erfindungsgemäßen Zusammensetzungen können nach Verfahren hergestellt werden, die dem Fachmann gut bekannt sind, insbesondere nach den Verfahren, die zur Herstellung von Öl-in-Wasser-Emulsionen oder Wasser-in-Öl-Emulsionen dienen.

Weitere Gegenstände der vorliegenden Erfindung sind Zubereitungen enthaltend die erfindungsgemäßen Partikel und einen oder mehrere kosmetisch, pharmazeutisch und/oder dermatologisch geeignete Träger, ein Verfahren zur Herstellung einer Zubereitung, welches dadurch gekennzeichnet ist, dass erfindungsgemäße Partikel mit einem kosmetisch pharmazeutisch und/oder dermatologisch geeigneten Träger vermischt werden, und die Verwendung von Partikeln zur Herstellung einer Zubereitung.

Diese Zusammensetzungen können insbesondere in Form von einfachen oder komplizierten Emulsionen (O/W, W/O, O/W/O oder W/O/W), wie Cremes, Milchen, Gelen oder Gel-Cremes, Pulvern und festen Stiften, vorliegen und gegebenenfalls können sie als Aerosole konfektioniert sein und in Form von Schäumen oder Sprays vorliegen. Vorzugsweise liegen diese Zusammensetzungen in Form einer O/W-Emulsion vor.

Die erfindungsgemäßen kosmetischen Zusammensetzungen können als Zusammensetzungen zum Schutz der menschlichen Epidermis oder der Haare gegen UV-Strahlung, als Sonnenschutzmittel oder Schminkprodukte verwendet werden.

Es soll darauf hingewiesen werden, dass in den erfindungsgemäßen Formulierungen zum Sonnenschutz, die einen Träger vom Typ einer Öl-in-Wasser-Emulsion aufweisen, die wässerige Phase (die insbesondere die hydrophilen Filter enthält) im Allgemeinen 50 bis 95 Gew.-% und vorzugsweise 70 bis 90 Gew.-%, bezogen auf die gesamte Formulierung, die Ölphase (die insbesondere die lipophilen Filter enthält) 5 bis 50 Gew.-% und vorzugsweise 10 bis 30 Gew.-%, bezogen auf die gesamte Formulierung und der (Co)emulgator oder die (Co)emulgatoren 0.5 bis 20 Gew.-% und vorzugsweise 2 bis 10 Gew.-%, bezogen auf die gesamte Formulierung, ausmachen.

Geeignet sind Zubereitungen für eine äußerliche Anwendung, beispielsweise als Creme, Lotion, Gel oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösevermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösemittel, Lösevermittler und Emulgatoren, z.B. Wasser, Ethanol, Isopropanol, Ethylcarbonat, Ethlyacetat, Benzylalkohol, Benzylbenzoat, Propylenglykol, 1.3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren. Salze von Fettsäurehalbestem, Fettsäureeiweißhydrolysaten, Isothionate. Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobemsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle, wie z.B. Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten

Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -Wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearen Silikonölen aufweisen oder vollständig aus solchen Ölen bestehen,
wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

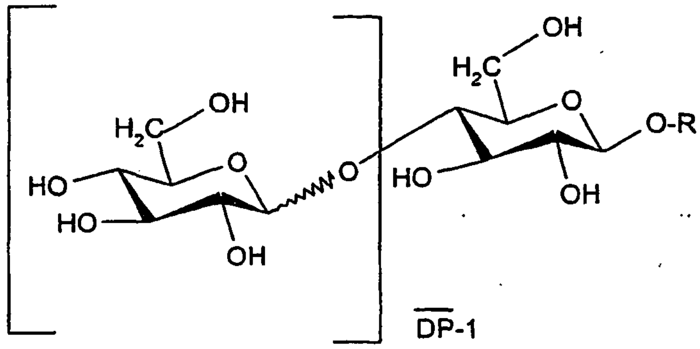

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \sum \frac{p_i}{100} \cdot i$$

Dabei stellen $p_1$, $p_2$, $p_3$ bis $p_i$ die Anteile der einfach, zweifach dreifach bis i-fach glucosylierten Produkte in Gewichtsprozenten dar. Erfindungsgemäß vorteilhaft werden Produkte mit Glucosylierungsgraden von 1-2, insbesondere vorteilhaft von 1. 1 bis 1.5. ganz besonders vorteilhaft von 1.2-1.4, insbesondere von 1.3 gewählt.

Der Wert $\overline{DP}$ trägt dem Umstande Rechnung, dass Alkylglucoside herstellungsbedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

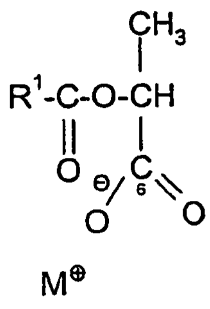

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der

Gesellschaft American Ingredients Company.

Die Betaine werden vorteilhaft gewählt aus der Gruppe der Substanzen, welche sich durch die Strukturformel

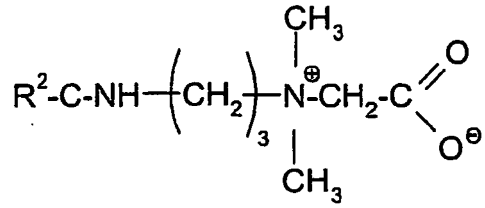

auszeichnen, wobei $R^2$ einen verzweigten oder unverzweigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0.01-20 Gew.-% bevorzugt 0.05-10 Gew.-%, besonders bevorzugt 0.1-5 Gew.-%, jeweils bezogen auf das Gesamtgewicht der Zusammensetzung, vorliegt oder vorliegen.

Zu Anwendung werden die erfindungsgemäßen, kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Als erfindungsgemäß besonders bevorzugter Emulgator für O/W-Emulsionen hat sich das Handelsprodukt Ceralution C der Firma Sasol erwiesen.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14.5-15.5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylen-glycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18) stearylether (Steareth-18), Polyethylenglycol(19)stearylether (Steareth-19), Polyethylenglycol(20)stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)-isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol (20)-isostearylether (Isosteareth-20), Polyethylenglycol(13)cetylether (Ceteth-13), Polyethylenglycol(14)cetylether (Ceteth-14), Polyethylenglycol(15)-cetylether (Ceteth-15), Polyethylenglycol(16)cetylether (Ceteth-16), Polyethylenglycol (17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14) isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol-(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)isolaurylether (Isolaureth-12), Polyethylenglycol(13)cetylstearylether (Ceteareth-13), Polymethylen-glycol(14)cetylstearylether (Ceteareth-14), Polyethylenglycol(15)cetyl-stearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21 )isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25) Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß.gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen UV-induzierte Alterungsprozesse sowie vor oxidativem Stress, d.h. gegen Schädigungen durch Radikale, wie sie z.B.

durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrig-alkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer der oder den Verbindungen der Formel I beispielsweise Fettalkohole, Fettsäuren, Fett-säureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder-polyole, wie Ethanol, Propylenglykol oder Glycerin, und ein Verdickungsmittel, wie

Kieselerde umfasst. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindem. In diesem Fall erfolgt geeignet eine Konfektionierung als Shampoo, Lotion, Gel oder Emulsion zum Ausspülen, wobei die jeweilige Zubereitung vor oder nach dem Shampoonieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösemittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die nachfolgenden Beispiele sollen die Erfindung näher erläutern, ohne sie jedoch zu beschränken.

Vergleichsbeispiele :

*Beispiel 1: Proben 1-4 mit Partikeldurchmesser von 100 nm*

a) Darstellung von 2-Hydroxy-4-(3-Triethoxysilylpropoxy)-diphenylketon Zu einer Mischung von 5.08 g (0.02 mol) 4-Allyloxy-2-hydroxybenzophenon und 3.28 g (0.02 mol) Triethoxysilan in 100 ml trockenem Toluol werden 10 Tropfen einer 5%-igen Lösung eines Vinyl-Siloxan-Komplex-Hydrosilylierungkatalysators in Toluol unter Stickstoffatmosphäre zugetropft. Die Reaktion ist nach ungefähr 30 Minuten beendet. Das Lösungsmittel wird bei 50˚C unter Vakuum entfernt und man erhält das Produkt als leicht gelbes viskoses Öl.

b) Co-Polymerisation
160 ml Ethanol und 83 ml demineralisiertes Wasser werden auf 58˚C erwärmt und mit 9.4 ml einer 25%-igen Ammoniaklösung (in Wasser) versetzt. Unter Rühren wird eine auf 58˚C erwärmte Mischung aus 20.94 g Tetraethoxysilan (TEOS) und 427 mg des in Schritt a) erhaltenen Produktes zu dem Ethanol/Wasser/Ammoniak-Gemisch zugegeben. Man lässt 15 Sekunden rühren und lässt dann die Reaktionsmischung bei 58˚C ohne Rühren 2 Stunden

stehen. Man erhält nach Abtrennung der Reaktionsmischung pulverförmige $SiO_2$-Monospheren mit 2-Hydroxy-4-(3-Triethoxysilylpropoxy)-diphenylketon im Kern der Partikel, wobei die Monospheren einen mittleren Durchmesser von 100nm aufweisen.

*Beispiel 2: Proben 5-6 mit Partikeldurchmesser von 250 nm*

a) siehe Beispiel 1a)

b) Co-Polymerisation

150 ml Ethanol und 56 ml demineralisiertes Wasser werden auf 60˚C erwärmt und mit 35 ml einer 25%-igen Ammoniaklösung (in Wasser) versetzt. Unter Rühren wird eine auf 60˚C erwärmte Mischung aus 20.94 g Tetraethoxysilan (TEOS) und 427 mg des in Schritt a) erhaltenen Produktes zu dem Ethanol/Wasser/Ammoniak-Gemisch zugegeben. Man lässt 15 Sekunden rühren und lässt dann die Reaktionsmischung bei 60˚C ohne Rühren 2 Stunden stehen. Man erhält nach Abtrennung der Reaktionsmischung pulverförmige $SiO_2$-Monospheren mit 2-Hydroxy-4-(3-Triethoxysilylpropoxy)-diphenylketon im Kern der Partikel, wobei die Monospheren einen mittleren Durchmesser von 250 nm aufweisen.

*Beispiel 3: Proben 7-8 mit Partikeldurchmesser von 500 nm*

a) siehe Beispiel 1a)

b) Co-Polymerisation

150 ml Ethanol und 56 ml demineralisiertes Wasser werden auf 30˚C erwärmt und mit 35 ml einer 25%-igen Ammoniaklösung (in Wasser) versetzt. Unter Rühren wird eine auf 30˚C erwärmte Mischung aus 20.94 g Tetraethoxysilan (TEOS) und 427 mg des in Schritt a) erhaltenen Produktes zu dem Ethanol/Wasser/Ammoniak-Gemisch zugegeben. Man lässt 15 Sekunden rühren und lässt dann die Reaktionsmischung bei 30˚C ohne Rühren 2 Stunden stehen. Man erhält nach Abtrennung der Reaktionsmischung pulverförmige $SiO_2$-Monospheren mit 2-Hydroxy-4-(3-Triethoxysilylpropoxy)-diphenylketon im Kern der Partikel, wobei die Monospheren einen mittleren Durchmesser von 500 nm aufweisen.

*Beispiel 4 gemäß vorliegender Erfindung: Probe 9-10 mit Partikeldurchmesser von 250 nm mit zusätzlicher Oberflächenfunktionatisierung*

a) siehe Beispiel 1a)

b) Co-Polymerisation und zusätzliche Funktionalisierung

150 ml Ethanol und 56 ml demineralisiertes Wasser werden auf 60˚C erwärmt und mit 35 ml einer 25%-igen Ammoniaklösung (in Wasser) versetzt. Unter Rühren wird eine auf 60˚C erwärmte Mischung aus 20.94 g Tetraethoxysilan (TEOS) und 427 mg des in Schritt a) erhaltenen Produktes zu dem Ethanol/Wasser/Ammoniak-Gemisch zugegeben. Man lässt 15 Sekunden rühren und lässt dann die Reaktionsmischung bei 60˚C ohne Rühren 2 Stunden stehen. Anschließend wird die Reaktion durch erneutes Rühren gestartet und noch nicht umgesetztes des in Schritt a) erhaltenen Produktes zur Reaktion gebracht. Es wird weitere 2.5 Stunden bei 60˚C gerührt. Man erhält nach Abtrennung der Reaktionsmischung pulverförmige $SiO_2$-Monospheren mit 2-Hydroxy-4-(3-Triethoxysilylpropoxy)-diphenylketon im Kern und auf der Oberfläche der Partikel, wobei die Monospheren einen mittleren Durchmesser von 250 nm aufweisen.

*Beispiel 5: Probe 11 mit Partikeldurchmesser von 250 nm mit zusätzlicher Oberflächenfunktionalisierung gemäß vorliegender Erfindung*

a) siehe Beispiel 1a)

b) Co-Polymerisation und zusätzliche Funktionalisierung

150 ml Ethanol und 56 ml demineralisiertes Wasser werden auf 60˚C erwärmt und mit 35 ml einer 25%-igen Ammoniaklösung (in Wasser) versetzt. Unter Rühren wird eine auf 60˚C erwärmte Mischung aus 20.94 g Tetraethoxysilan (TEOS) und 427 mg des in Schritt a) erhaltenen Produktes zu dem Ethanol/Wasser/Ammoniak-Gemisch zugegeben. Man lässt 15 Sekunden rühren und lässt dann die Reaktionsmischung bei 60˚C ohne Rühren 2 Stunden stehen. Anschließend wird erneut gerührt und 850 mg des in Schritt a) erhaltenen Produktes (gelöst in 100 ml Ethanol) tropfenweise innerhalb einer Stunde zu der Suspension zugegeben. Es wird weitere 2 Stunden bei 60˚C gerührt. Man erhält nach Abtrennung der Reaktionsmischung pulverförmige $SiO_2$-Monospheren mit 2-Hydroxy-4-(3-Triethoxysilylpropoxy)-diphenylketon im Kern und auf der Oberfläche der Partikel, wobei die Monospheren einen mittleren Durchmesser von 250 nm aufweisen.

*UV-Messungen:*

50 mg der jeweiligen Probe werden in einem 100 ml-Messkolben in 50 ml 2-Propanol unter Zuhilfenahme eines Ultraschallbades suspendiert. Der Messkolben wird mit 2-Propanol auf 100 ml aufgefüllt und die Suspension erneut homogenisiert. Eine Probe der Lösung wird im UV-Spektrometer vermessen (Typ: Perkin Elmer Lambda 900 mit Ulbrichtkugel (150 mm Durchmesser)).

Die Ergebnisse der UV-spektrometrischen Untersuchungen der gemäß den Beispielen 1 bis 5 erhaltenen Proben sind in Tabelle 1 zusammengefasst.

Tabelle 1: Übersicht über Proben 1 bis 11 und der Vergleichsproben

| **Probe** | **Ort der Funktionalisierung** | **Mittlerer Partikeldurchmesser [nm]** | **Anteil der org. Verbindung [mmol/g]** | **Molarer Extinktionskoeffizient ε [$Lmol^{-1}cm^{-1}$]** |
|---|---|---|---|---|
| 1 | Kern | 100 | 0.12 | 9588 |
| 2 | Kern | 100 | 0.22 | 10269 |
| 3 | Kern | 100 | 0.32 | 9086 |
| 4 | Kern | 100 | 0.41 | 8933 |
| 5 | Kern | 250 | 0.21 | 9628 |
| 6 | Kern | 250 | 0.313 | 8907 |
| 7 | Kern | 500 | 0.10 | 7989 |
| 8 | Kern | 500 | 0.21 | 8751 |
| 9 | Kern + Oberfläche | 250 | 0.02 | 46456 |
| 10 | Kern + Oberfläche | 250 | 0.18 | 10037 |
| 11 | Kern + Oberfläche | 250 | 0.27 | 7804 |
| Vergleich 1[a] | Oberfläche | 250 | 0.12[b] | 6795 |
| Vergleich 2[a] | Oberfläche | 500 | 0.14[b] | 7163 |
| [a] Partikel gemäß EP 1 205 177<br>[b] Anteil an der Oberfläche | | | | |

Die erfindungsgemäßen Partikel weisen höhere molare Extinktionskoeffizienten auf, das heißt, sie weisen bessere Lichtschutzeigenschaften auf, als die entsprechenden Vergleichsproben gemäß dem Stand der Technik.

*Photostabilitätsmessungen:*

Proben werden unter Verwendung eines Suntest CPS mit Xenonstrahler (Strahlung begrenzt auf Wellenlängen ≥ 290 nm) für 130 min mit einer Leistung von 89.6 $W/m^2$ bestrahlt. Die UV-Spektren dem bestrahlten Proben werden mit denen von unbestrahlten Proben verglichen. Tabelle 2 faßt die Ergebnisse für die UV-Aktivität der Proben nach Bestrahlung zusammen.

Tabelle 2. Photostabilität einzelner Proben nach Bestrahlung in (%)

| UV-Region | Benzophenon-3 | Probe 5 | Vergleich 1[a] |
|---|---|---|---|
| UV (290-400 nm) | 79 ± 3 | 93 ± 1 | 89 ± 1 |
| UVA (320-400 nm) | 80 ± 3 | 95 ± 1 | 88 ± 1 |
| UVB (290-320 nm) | 77 ± 2 | 89 ± 1 | 89 ± 2 |
| [a] Partikel gemäß EP 1 205 177 | | | |

Benzophenon-3 ist zu 80 % photostabil. Die erfindungsgemäßen Proben (Probe 5) zeigen eine verbesserte Photostabilität, die auch besser als bei auf der Oberfläche funktionalisierten Proben gemäß dem Stand der Technik ist (Vergleich 1).

***Beispiele für Hautschutzformulierungen***

**Hautschutzlotion (O/W)**

| **Rohstoff** | **INCI** | **Gew.%** | |
|---|---|---|---|
| **A** Partikel aus einem der Beispiele 4 bis 5 | | | 5,00 |
| Emulgator E 2155 | Stearyl Alcohol (and) Steareth-7 (and) Steareth-10 | (2) | 3,00 |
| Teginacid H | Glyceryl Stearate (and) Cateth-20 | (2) | 3,00 |
| Imwitor 900 | Glyceryl Stearate | (4) | 3,00 |
| Lunacera M | Microwax | (6) | 1,00 |
| Luvitol EHO | Cetearyl Octanoate | (3) | 11,50 |
| Cetiol | Oleyl Oleate | (5) | 6,00 |
| Miglyol 812 Neutralöl | Caprylic/Capric Triglyceride | (4) | 6,00 |
| | | | |
| **B** Propandiol-1,2 | Propylene Gylcol | (1) | 4,00 |
| Allantoin | Allantoin | (1) | 0,20 |
| Konservierungsmittel | | | q.s. |
| Wasser, demineralisiert | Aqua | | ad 100,00 |

Herstellung:

Die Phase A wird auf 75˚C und die Phase B auf 80˚C erwärmt. Die Phase B wird unter Rühren langsam zu der Phase A gegeben. Das Gemisch wird homogenisiert und unter Rühren abgekühlt.

Bemerkungen:

Viskosität 9200 mPas (Brookfield RVT, Sp. C, 10 Upm) bei 24˚C

$pH_{24^\circ C}$=5,0
Konservierungsmittel:
0,05% Propyl-4-hydroxybenzoat
0,15% Methyl-4-hydroxybenzoat

Bezugsquellen:

(1) Merck KGaA, Darmstadt
(2) Th. Goldschmidt, Essen
(3) BASF, Ludwigshafen
(4) Hüls, Troisdorf AG, Witten
(5) Henkel, Düsseldorf
(6) H.B. Fuller GmbH, Lüneburg

**Hautschutzlotion (O/W)**

| **Rohstoff** | **INCI** | **Gew.%** | |
|---|---|---|---|
| **A** Partikel aus einem der Beispiele 4 bis 5 | | | 1,00 |
| Emulgator E 2155 | Stearyl Alcohol (and) Steareth-7 (and) Steareth-10 | (2) | 3,00 |
| Teginacid H | Glyceryl Stearate (and) Cateth-20 | (2) | 3,00 |
| Imwitor 900 | Glyceryl Stearate | (4) | 3,00 |
| Lunacera M | Microwax | (6) | 1,00 |
| Luvitol EHO | Cetearyl Octanoate | (3) | 11,50 |
| Cetiol | Oleyl Oleate | (5) | 7,00 |
| Miglyol 812 Neutralöl | Caprylic/Capric Triglyceride | (4) | 7,00 |
| | | | |
| **B** Propandiol-1,2 | Propylene Gylcol | (1) | 4,00 |
| Allantoin | Allantoin | (1) | 0,20 |
| Konservierungsmittel | | | q.s. |
| Wasser, demineralisiert | Aqua | | ad 100,00 |

Herstellung:

Die Phase A wird auf 75˚C und die Phase B auf 80˚C erwärmt. Die Phase B wird unter Rühren langsam zu der Phase A gegeben. Das Gemisch wird homogenisiert und unter Rühren abgekühlt.

Bemerkungen:

Konservierungsmittel:

0,05% Propyl-4-hydroxybenzoat
0,15% Methyl-4-hydroxybenzoat

Bezugsquellen:

(1) Merck KGaA, Darmstadt
(2) Th. Goldschmidt, Essen
(3) BASF, Ludwigshafen
(4) Hüls, Troisdorf AG, Witten
(5) Henkel, Düsseldorf

(6) H.B. Fuller GmbH, Lüneburg

***Beispiele für Sonnenschutzformulierungen***

**Sonnenschutzlotion mit IR3535™ (O/W)**

| Rohstoff | INCI | Gew.% | |
|---|---|---|---|
| A Partikel aus einem der Beispiele 4 bis 5 | | | 3,00 |
| Eusolex 6300 | 4-Methylbenzylidene Camphor | (1) | 3,00 |
| IR 3535™ | Ethyl Butylacetylaminopropionate | (1) | 10,00 |
| (-)-?-Bisabolol | Bisabolol | (1) | 0,30 |
| Montanov 68 | Cetearyl Alcohol (and) Cetearyl Glucoside | (2) | 4,00 |
| Myritol 312 | Caprylic/Capric Triglyceride | (3) | 2,00 |
| Mirasil CM 5 | Cyclomethicone | (4) | 2,00 |
| Mirasil DM 350 | Dimethicone | (4) | 1,00 |
| | | | |
| B Wasser, demineralisiert | Aqua | | ad 100 |
| Glycerin, 87%ig | Glycerin | (1) | 3,00 |
| Konservierungsmittel | | | q.s. |
| | | | |
| C Rhodicare S | Xanthan Gum | (4) | 0,50 |

Herstellung:

Die Phasen A und B werden getrennt voneinander auf 75°C erwärmt. Die Phase C wird bei 75°C unter Rühren langsam zu der Phase B gegeben. Das Gemisch wird gerührt, bis es homogen ist. Anschließend wird die Phase A zu dem Gemisch gegeben. Das Gemisch wird gerührt, bis es homogen ist, und dann unter Rühren abgekühlt.

Bemerkungen:

Konservierungsmittel:

0,05% Propyl-4-hydroxybenzoat
0,15% Methyl-4-hydroxybenzoat
0,30% Germall 115 (ISP, Frechen)

Bezugsquellen:

(1) Merck KgaA, Darmstadt
(2) Interorgana, Köln
(3) Henkel, KgaA, Düsseldorf
(4) Rhodia, Frankfurt

Sonnenschutzmilch (O/W)

| Rohstoff | INCI | Gew.% | |
|---|---|---|---|
| **A** Partikel aus einem der Beispiele 4 bis 5 | | | 4,00 |
| Eusolex 6300 | 4-Methylbenzylidene Camphor | (1) | 4,00 |
| Emulgator E 2155 | Stearyl Alcohol (and) Steareth-7 (and) Steareth-10 | (2) | 3,00 |
| Teginacid H | Glyceryl Stearate (and) Cateth-20 | (2) | 2,00 |
| Luvitol EHO | Cetearyl Octanoate | (3) | 14,00 |
| Imwitor 900 | Glyceryl Stearate | (4) | 3,00 |
| Cetiol | Oleyl Oleate | (5) | 6,00 |
| Luncacera M | Mikrowax | (6) | 1,00 |
| Miglyol 812 Neutralöl | Caprylic/Capric Triglyceride | (4) | 4,00 |
| | | | |
| **B** Eusolex 232 | Phenylbenzimidazole Sulfonic Acid | (1) | 2,00 |
| Tris(hydroxymethyl)-aminomethan | Tromethamine | (1) | 1,07 |
| Propandiol-1,2 | Propylene Glycol | (1) | 4,00 |
| Allantoin | Allantoin | (1) | 0,20 |
| Konservierungsmittel | | | q.s. |
| Wasser, demineralisiert | Aqua | | ad 100,00 |
| | | | |
| **C** Carbopol ETD 2050 | Carbomer ETD 2050 | (7) | 0,25 |
| Wasser, demineralisiert | Aqua | | 30,00 |
| | | | |
| **D** Tris(hydroxymethyl)-aminomethan | Tromethamine | (1) | 0,25 |
| Wasser, demineralisiert | Aqua | | 4,00 |

Herstellung:

Carbopol ETD 2050 wird in Wasser homogen dispergiert, um die Phase C zu erhalten. Die Phase D wird dann unter Homogenisieren in die Phase C eingetragen. Zur Neutralisierung von Eusolex 232 wird das Tris(hydroxymethyl)-aminomethan im Wasser der Phase B gelöst, und Eusolex 232 wird unter Rühren zugegeben. Nach vollständiger Lösung werden die restlichen Inhaltsstoffe der Phase B zugegeben, und die Phase B wird langsam unter Homogenisieren in die Phasen C/D eingetragen. Die Phase A wird unter Erhitzen gelöst und langsam unter Homogenisieren zugegeben.

Bemerkungen:

Konservierungsmittel:

0,05% Propyl-4-hydroxybenzoat
0,15% Methyl-4-hydroxybenzoat

Bezugsquellen:

(1) Merck KGaA, Darmstadt
(2) Th. Goldschmidt, Essen
(3) BASF, Ludwigshafen
(4) Hüls, Troisdorf AG, Witten
(5) Henkel, Düsseldorf
(6) H.B. Fuller GmbH, Lüneburg
(7) Goodrich, Neuss

**Sonnenschutzlotion (W/O)**

| Rohstoff | | Gew.% |
|---|---|---|
| **A** Partikel aus einem der Beispiele 4 bis 5 | (1) | 5,00 |
| Eusolex HMS | (1) | 5,00 |
| Eusolex OS | (1) | 5,00 |
| Eusolex OCR | (1) | 5,00 |
| Abil WE 09 | (2) | 5,00 |
| Jojobaöl | (3) | 3,00 |
| Cetiol V | (4) | 3,00 |
| Prisorine 2021 | (5) | 2,00 |
| Lunacera M | (6) | 1,80 |
| Miglyol 812 Neutralöl | (6) | 3,00 |
| | | |
| **B** Glycerin (etwa 87%) | (1) | 2,00 |
| Natriumchlorid | (1) | 0,40 |
| Konservierungsmittel | (1) | q.s. |
| Wasser, demineralisiert | | ad 100,00 |

Herstellung:

Die Phase B wird auf 80°C und die Phase A auf 75°C erwärmt. Die Phase B wird langsam in die Phase A eingerührt. Das Gemisch wird homogenisiert und unter Rühren abgekühlt.

Bemerkungen:

Konservierungsmittel:

0,05% Propyl-4-hydroxybenzoat
0,15% Methyl-4-hydroxybenzoat

Bezugsquellen:

(1) Merck KGaA, Darmstadt

(1) Th. Goldschmidt AG, Essen
(2) H. Lamotte, Bremen
(3) Henkel KGaA, Düsseldorf

(4) Unichema, Emmerich
(5) H.B. Fuller, Lüneburg
(6) Hüls Troisdorf AG, Witten

<u>**Sonnenschutzcreme (W/O)**</u>

| Rohstoff | INCI | Gew.% | |
|---|---|---|---|
| **A** Partikel aus einem der Beispiele 4 bis 5 | | (1) | 2,50 |
| Eusolex T-2000 | mikron. Titandioxid | (1) | 2,50 |
| Eusolex 6300 | 4-Methylbenzylidene Camphor | (1) | 2,00 |
| Dehymuls E | Dicocoyl Pentyerythrityl Citrate (and) Sorbitan Sesquioleate (and) Cera Alba (and) Aluminium Stearate | (2) | 6.00 |
| Arlacel 989 | PEG-7 Hydrogenated Castor Oil | (3) | 1,00 |
| Bienenwachs | Cera Alba | (1) | 2,00 |
| Zinkstearat | Zinc Stearate | (1) | 2,00 |
| Cetiol J 600 | Oleyl Erucate | (2) | 6,00 |
| Cetiol V | Decyl Oleate | (2) | 6,00 |
| Cetiol OE | Dicaprylyl Ether | (2) | 5,00 |
| Dow Corning 200 (100 cs) | Dimethicone | (4) | 1,00 |
| DL-?-Tocopherolacetat | Tocopheryl Acetate | (1) | 1,00 |
| Vitamin-A-palmitat | Retinyl Palmitate | (5) | 0,50 |
| | | | |
| **B** Eusolex 232 | Phenylbenzimidazole Sulfonic Acid | (1) | 2,00 |
| Tris(hydroxymethy)- aminomethan | Tromethamine | (1) | 0,88 |
| Glycerin (etwa 87%) | Glycerin | (1) | 5,00 |
| Magnesiumsulfat Heptahydrat | Magnesium Sulfate | (1) | 1,00 |
| Allantoin | Allantoin | (1) | 0,20 |
| Konservierungsmittel | | | q.s. |
| Wasser, demineralisiert | | | ad 100,00 |

<u>Herstellung:</u>

Zur Neutralisierung von Eusolex 232 wird das Tris(hydroxymethyl)-aminomethan im Wasser der Phase B gelöst, und Eusolex 232 wird unter Rühren zugegeben. Nach vollständiger Lösung werden die restlichen Rohstoffe der Phase B zugegeben und auf 80˚C erwärmt. Die Phase A wird auf 75˚C erwärmt. Die Phase B wird langsam in Phase A eingerührt, und das Gemisch wird unter Rühren abgekühlt.

<u>Bemerkungen:</u>

Konservierungsmittel:

0,05% Propyl-4-hydroxybenzoat
0,15% Methyl-4-hydroxybenzoat

Bezugsquellen:

(1) Merck KGaA, Darmstadt
(2) Henkel KGaA, Düsseldorf
(3) ICI, Essen
(4) Dow Coming, Düsseldorf
(5) Hoffmann La Roche, Schweiz

**Sonnenschutzgel (O/W) mit UV-A/B-Schutz**

| Rohstoff | | Gew.% |
|---|---|---|
| **A** Partikel aus einem der Beispiele 4 bis 5 | (1) | 2,00 |
| Eusolex 2292 | (1) | 5,50 |
| Oxynex K flüssig | (1) | 1,00 |
| Luvitol EHO | (2) | 9,00 |
| Dow Corning 200 (100 cs) | (3) | 2,00 |
| Antaron V-220 | (4) | 2,00 |
| Jojobaöl | (5) | 5,00 |
| | | |
| **B** Tris(hydroxymethyl)-aminomethan | (1) | 0,60 |
| Konservierungsmittel | | q.s. |
| Wasser, demineralisiert | | ad 100,00 |
| | | |
| **C** Pemulen TR-1 | (6) | 0,50 |
| Wasser, demineralisiert | | 29,50 |
| | | |
| **D** Aloe Vera Gel 1:10 | (7) | 1,00 |

Herstellung:

Das PemulenTR-1 wird im Wasser homogen dispergiert und vorgequollen, um die Phase C zu erhalten. Die Phase B wird unter Homogenisieren in die Phase C eingetragen. Die Phase A wird unter Erwärmen gelöst und langsam unter Homogenisieren zugegeben. Bei 35˚C wird die Phase D zugesetzt und nochmals homogenisiert.

Bemerkungen:

Konservierungsmittel:

0,05% Propyl-4-hydroxybenzoat
0,15% Methyl-4-hydrocybenzoat

Bezugsquellen:

(1) Merck kGaA, Darmstadt
(2) BASF, Ludwigshafen
(3) Dow Coming, Düsseldorf

(4) GAF, Frechen
(5) Henry Lamotte, Bremen
(6) Goodrich, Neuss
(7) Galke, Gittelde

**Sonnenschutzspray (O/W)**

| Rohstoff | INCI | Gew.% | |
|---|---|---|---|
| **A** Partikel aus einem der Beispiele 4 bis 5 | | (1) | 3,00 |
| Eusolex 2292 | Octyl Methoxycinnamate | (1) | 7,50 |
| Eusolex HMS | Homosalate | (1) | 7,00 |
| Volpo S-2 | Steareth-2 | (2) | 0,40 |
| Volpo S-10 | Steareth-10 | (2) | 0,80 |
| Pemulen TR-2 | Acrylate/C 10-30 Alkyl Acrylate Crosspolymer | (3) | 0,18 |
| Hetester PHA | Propylene Glycol Isoceteth-3 Acetate | (4) | 5,00 |
| Performa V 825 | Synthetic Wax | (5) | 0,80 |
| Dow Corning 200 (100cs) | Dimethicone | (6) | 1,00 |
| Oxynex K flüssig | PEG-8 (and) Tocopherol (and) Ascorbyl Palmitate (and) Ascorbic Acid (and) Citric Acid | (1) | 0,10 |
| | | | |
| **B** Eusolex 232 | Phenylbenzimidazole Sulfonic Acid | (1) | 1,00 |
| Triethanolamin | Triethanolamine | (1) | 0,90 |
| Propandiol-1,2 | Propylene Glycol | (1) | 2,00 |
| Konservierungsmittel | | | q.s. |
| Wasser, demineralisiert | Aqua | | ad 100,00 |

Herstellung:

Zur Neutralisierung von Eusolex 232 wird das Triethanolamin ins Wasser der Phase B gegeben, und Eusolex 232 wird unter Rühren zugegeben. Nach vollständiger Lösung werden die restlichen Rohstoffe der Phase B zugegeben und auf 80°C erwärmt. Die Phase A wird bis auf das Pemulen zusammengegeben und auf 80°C erwärmt. Dann wird das Pemulen in Phase A eingerührt. Die Phase B wird unter Rühren langsam zu der Phase A gegeben, und das Gemisch wird homogenisiert und unter Rühren abgekühlt.

Bemerkungen:

Konservierungsmittel:

0,05% Propyl-4-hydroxybenzoat
0,15% Methyl-4-hydroxybenzoat

Bezugsquellen:

(1) Merck KGaA, Darmstadt
(2) Croda, Nettetal
(3) Goodrich, Neuss

(4) ROVI, Schlüchtern
(5) New Phase, NJ 08554
(6) Dow Coming, Wiesbaden

## Patentansprüche

1. Partikel umfassend ein anorganisches Netzwerk, welches ein Oxid, Oxidhydrat, Phosphat, Carbonat, Sulfat oder Nitrid ist, und über eine Spacer-Gruppe kovalent an das Netzwerk gebundene organische Verbindungen, **dadurch gekennzeichnet, dass** die organischen Verbindungen, ausgewählt aus der Gruppe aus Dibenzoylmethanderivaten, Aminobenzoesäure, Zimtsäure, Salicylsäure, Benzylidenkampfer, Phenylbenzimidazol, Diphenylacrylat, Triazin, Benzophenon, Diarylbutadien und vinylgruppenhaltigen Amiden, im Inneren der Partikel vorliegen und die Oberfläche der Partikel mit der gleichen, der im Inneren der Partikel vorliegenden organischen Verbindung, und/oder davon verschiedenen organischen Verbindungen ausgewählt aus der Gruppe der lichtabsorbierenden organischen Verbindungen, der Verbindungen mit antioxidanten und/oder radikalinhibierenden Eigenschaften, der Repellentien und der Konservierungsmittel durch kovalente Anbindung funktionalisiert ist und
wobei die Spacer-Gruppe die allgemeine Formel

$$(-A)_aMe(R^1)_b(R^2)_c-X-$$

aufweist, wobei
A = O, S, NH bedeutet,
Me = Si, Al, Ti, Zr bedeutet,
$R^1$ und $R^2$ gleich oder verschieden sein können und eine geradkettige oder verzweigte Alkyl-, Alkoxy-, Halogen- oder Hydroxygruppe bedeuten
a 1 bis 3 ist,
b und c 0-2 sein können, mit der Bedingung, dass für Me = Si, Ti, Zr a+b+c=3 ist und für Me = Al a+b+c=2 ist und
X = eine geradkettige oder verzweigte Alkylgruppe mit bis zu 20, vorzugsweise mit 1 bis 12 und besonders bevorzugt mit 3 bis 12 Kohlenstoffatomen bedeutet, wobei die Spacer-Gruppe über den Substituenten $(-A)_a$ an das anorganische Netzwerk gebunden ist.

2. Partikel gemäß Anspruch 1, **dadurch gekennzeichnet, dass** es sich um sphärische Partikel handelt

3. Partikel nach Anspruch 1 oder 2, **dadurch gekennzeichnet, dass** die Partikel eine Größe von 1 nm bis 250 $\mu$m besitzen.

4. Partikel nach einem der Ansprüche 1 bis 3, **dadurch gekennzeichnet, dass** das Oxid, Magnesiumoxid, Aluminiumoxid, Siliziumoxid, Zinkoxid, Ceroxid, Titanoxid, Zirkoniumoxid, Manganoxid, Boroxid, Eisenoxid oder ein Gemisch hieraus ist.

5. Partikel nach Anspruch 4, **dadurch gekennzeichnet, dass** das anorganische Netzwerk Siliziumoxid ist,

6. Partikel nach einem der Ansprüche 1 bis 5, **dadurch gekennzeichnet, dass** die lichtabsorbierende Verbindung ausgewählt ist aus der Gruppe aus Dibenzoylmethanderivaten, Aminobenzoesäure, Zimtsäure, Salicylsäure, Benzylidenkampfer, Phenylbenzimidazol, Diphenylacrylat, Triazin, Benzophenon, Diarylbutadien und vinylgruppenhaltigen Amiden.

7. Partikel nach einem der Ansprüche 1 bis 5, **dadurch gekennzeichnet, dass** die Verbindungen mit antioxidanten und/oder radikalinhibierenden Eigenschaften aus Flavonoiden, Coumaranonen, Vitaminen und BHT ausgewählt sind.

8. Partikel nach einem der Ansprüche 1 bis 5, **dadurch gekennzeichnet, dass** die Repellentien ausgewählt sind aus Amiden und/oder Derivaten davon.

9. Partikel gemäß einem der Ansprüche 1 bis 5, **dadurch gekennzeichnet, dass** das Konservierungsmittel Benzalkoniumchlorid, Benzoesäure und deren Salze, Cetylpyridiniumchlorid, Cetrimoniumchlorid sowie Salicylsäure und deren Salze ist.

**10.** Verfahren zur Herstellung von Partikeln gemäß Anspruch 1, umfassend

a) das Umsetzen einer organischen Verbindung ausgewählt aus der Gruppe aus Dibenzoylmethanderivaten, Aminobenzoesäure, Zimtsäure, Salicylsäure, Benzylidenkampfer, Phenylbenzimidazol, Diphenylacrylat, Triazin, Benzophenon, Diarylbutadien und vinylgruppenhaltigen Amiden, unter Ausbildung einer kovalenten Bindung mit einer Substanz mit mindestens zwei durch eine Spacer-Gruppe getrennten reaktiven Gruppen der allgemeinen Formel

$$(R^3A)_aMe(R^1)_b(R^2)_c\text{-}X\text{-}Y,$$

wobei
A = O S, NH bedeutet
Me = Si, Al, Ti, Zr bedeutet
$R^1$ und $R^2$ gleich oder verschieden sein können und eine geradkettige oder verzweigte Alkyl-, Alkoxy-, Halogen- oder Hydroxygruppe bedeuten.
$R^3$ = Wasserstoff oder eine geradkettige oder verzweigte Alkygruppe bedeutet
a = 1 bis 3 ist
b und c 0-2 sein können, mit der Bedingung, dass für Me = Si, Ti, Zr a+b+c=3 ist und für Me Al a+b+c=2 ist, und
X = eine geradkettige oder verzweigte Alkylgruppe mit bis zu 20, vorzugsweise mit 1 bis 12 und besonders bevorzugt mit 3 bis 12 Kohlenstoffatomen bedeutet, und
Y = geradkettiges oder verzweigtes Alkenyl, Alkinyl, Alkoxy, SH, $NH_2$, Carboxy, Halogen oder Hydroxy bedeutet, wobei die kovalente Bindung zwischen der organischen Verbindung und der Spacer-Gruppe unter Abspaltung oder über Verknüpfung mit der reaktiven Gruppe Y ausgebildet wird und
b) die Co-Polymerisation der in a) erhaltenen Verbindung mit einem Precursor, der für den Aufbau eines anorganischen Netzwerks, welches ein Oxid, Oxidhydrat, Phosphat, Carbonat, Sulfat oder Nitrid ist, geeignet ist, wobei die zusätzliche Oberflächenfunktionalisierung durch Umsetzung der erhaltenen Partikel mit der gleichen, der im Inneren der Partikel vorliegenden organischen Verbindung, und/oder davon verschiedenen organischen Verbindungen ausgewählt aus der Gruppe der lichtabsorbierenden organischen Verbindungen, der Verbindungen mit antioxidanten und/oder radikalinhibierenden Eigenschaften, der Repellentien und der Konservierungsmittel erfolgt.

**11.** Verfahren gemäß Anspruch 10, **dadurch gekennzeichnet, dass** es sich bei Schritt b) um eine hydrolytische Polykondensation handelt.

**12.** Verfahren gemäß Anspruch 10 oder 11, **dadurch gekennzeichnet, dass** als Precursor Hydroxide, Halogenide, Alkoxide oder andere hydrolisierbare Gruppen enthaltende Verbindungen der Elemente Magnesium, Aluminium, Silizium, Zink, Cer, Titan, Zirkonium, Mangan, Bor, Eisen und/oder Gemische hieraus eingesetzt werden.

**13.** Verfahren gemäß Anspruch 12, **dadurch gekennzeichnet, dass** es sich bei dem Precursor um Alkoxide des Siliziums handelt

**14.** Verfahren gemäß einem der Ansprüche 10 bis 13, **dadurch gekennzeichnet, dass** die Oberfläche der Partikel zusätzlich mit den gemäß a) erhaltenen Verbindungen funktionalisiert wird.

**15.** Verwendung von Partikeln gemäß Anspruch 1 in Formulierungen und Zubereitungen.

**16.** Zubereitungen enthaltend Partikel gemäß Anspruch 1 und mindestens einen kosmetisch, pharmazeutisch und/oder dermatologisch verträglichen Träger und/oder Hilfsstoff.

**17.** Zubereitungen gemäß Anspruch 16, **dadurch gekennzeichnet, dass** es sich um Zubereitungen mit Lichtschutzeigenschaften handelt.

**18.** Zubereitungen gemäß Anspruch 16 oder 17, **dadurch gekennzeichnet, dass** es sich um topisch anwendbare Zubereitungen handelt.

**19.** Zubereitungen nach mindestens einem der vorstehenden Ansprüche, **dadurch gekennzeichnet, dass** die Zubereitungen zusätzlich mindestens einen organischen und/oder anorganischen UV-Filter enthalten.

**20.** Zubereitungen nach mindestens einem der vorstehenden Ansprüche, **dadurch gekennzeichnet, dass** die Zubereitung mindestens einen Selbstbräuner enthalten.

**21.** Zubereitungen nach mindestens einem der vorhergehenden Ansprüche, **dadurch gekennzeichnet, dass** die Zubereitungen mindestens einen Photostabilisator enthalten.

**22.** Zubereitungen nach Anspruch 21, **dadurch gekennzeichnet, dass** der Photostabilisator ausgewählt ist aus Verbindungen entsprechend der Formel V

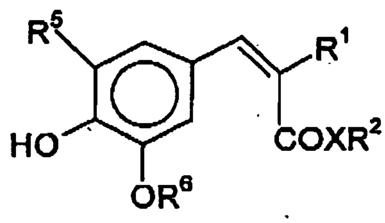

V,

wobei
$R^1$ ausgewählt ist aus $-C(O)CH_3$, $-CO_2R^3$, $-C(O)NH_2$ and $-C(O)N(R^4)_2$; X is O or NH;
$R^2$ steht für einen linearen oder verzweigten $C_{1-30}$Alkylrest;
$R^2$ steht für einen linearen oder verzweigten $C_{1-20}$-Alkylrest,
alle $R^4$ unabhängig voneinander stehen für H oder lineare oder verzweigte $C_{1-8}$-Alkylreste
$R^5$ steht für H, einen linearen oder verzweigten $C_{1-8}$-Alkylrest oder einen linearen oder verzweigten $-O-C_{1-8}$-Alkylrest und
$R^6$ steht für einen $C_{1-8}$-Alkylrest.

**23.** Zubereitungen nach mindestens einem der vorhergehenden Ansprüche, **dadurch gekennzeichnet, dass** die Zubereitungen einen oder mehrere weitere UV-Filter enthalten, die ausgewählt sind aus der Gruppe bestehend aus 3-(4'-Methylbenzyliden)-dl-kampfer, Methoxyzimtsäureoctylester, 3,3,5-Trimethyl-cyclohexylsalicylat, 4-(Dimethylamino)benzoesäure2-ethylhexylester, 2-Cyano-3,3-diphenylacrylsäure-2-ethylhexylester, 2-Phenylbenzimidazol-5-sulfonsäure sowie ihren Kalium-, Natrium- und Triethanolaminsalzen.

**24.** Zubereitungen nach mindestens einem der vorhergehenden Ansprüche geeignet zum Schutz von Körperzellen gegen oxidativen Stress, **dadurch gekennzeichnet, dass** sie vorzugsweise ein oder mehrere Antioxidantien enthalten.

## Claims

**1.** Particles comprising an inorganic network which is an oxide, oxide hydrate, phosphate, carbonate, sulfate or nitride and organic compounds covalently bonded to the network via a spacer group, **characterised in that** the organic compounds, selected from the group comprising dibenzoylmethane derivatives, aminobenzoic acid, cinnamic acid, salicylic acid, benzylidenecamphor, phenylbenzimidazole, diphenyl acrylate, triazine, benzophenone, diarylbutadiene and vinyl-containing amides, are present in the interior of the particles, and the surface of the particles has been functionalised with the same organic compound present in the interior of the particles and/or organic compounds which are different therefrom selected from the group of the light-absorbent organic compounds, compounds having antioxidant and/or free-radical-inhibiting properties, repellents and preservatives by covalent bonding, and where the spacer group has the general formula

$$(-A)_aMe(R^1)_b(R^2)_c\text{-}X\text{-}$$

where
A = O, S, NH,
Me = Si, Al, Ti, Zr,
$R^1$ and $R^2$ may be identical or different and denote a straight-chain or branched alkyl, alkoxy, halogen or hydroxyl group
a is 1 to 3,

b and c may be 0-2, with the condition that a+b+c = 3 for Me = Si, Ti, Zr and a+b+c = 2 for Me = Al and
X = a straight-chain or branched alkyl group having up to 20, preferably having 1 to 12 and particularly preferably having 3 to 12, carbon atoms, where the spacer group is bonded to the inorganic network via the substituent $(-A)_a$.

**2.** Particles according to Claim 1, **characterised in that** they are spherical particles.

**3.** Particles according to Claim 1 or 2, **characterised in that** the particles have a size of 1 nm to 250 μm.

**4.** Particles according to one of Claims 1 to 3, **characterised in that** the oxide is magnesium oxide, aluminium oxide, silicon oxide, zinc oxide, cerium oxide, titanium oxide, zirconium oxide, manganese oxide, boron oxide, iron oxide or a mixture thereof.

**5.** Particles according to Claim 4, **characterised in that** the inorganic network is silicon oxide.

**6.** Particles according to one of Claims 1 to 5, **characterised in that** the light-absorbent compound is selected from the group comprising dibenzoylmethane derivatives, aminobenzoic acid, cinnamic acid, salicylic acid, benzylidenecamphor, phenylbenzimidazole, diphenyl acrylate, triazine, benzophenone, diarylbutadiene and vinyl-containing amides.

**7.** Particles according to one of Claims 1 to 5, **characterised in that** the compounds having antioxidant and/or free-radical-inhibiting properties are selected from flavonoids, coumaranones, vitamins and BHT.

**8.** Particles according to one of Claims 1 to 5, **characterised in that** the repellents are selected from amides and/or derivatives thereof.

**9.** Particles according to one of Claims 1 to 5, **characterised in that** the preservative is benzalkonium chloride, benzoic acid and salts thereof, cetylpyridinium chloride, cetrimonium chloride as well as salicylic acid and salts thereof.

**10.** Process for the production of particles according to Claim 1, comprising

a) the reaction of an organic compound selected from the group comprising dibenzoylmethane derivatives, aminobenzoic acid, cinnamic acid, salicylic acid, benzylidenecamphor, phenylbenzimidazole, diphenyl acrylate, triazine, benzophenone, diarylbutadiene and vinyl-containing amides with a substance having at least two reactive groups separated by a spacer group of the general formula

$$(R^3A)_aMe(R^1)_b(R^2)_c\text{-}X\text{-}Y,$$

where
A=O, S, NH
Me = Si, Al, Ti, Zr
$R^1$ and $R^2$ may be identical or different and denote a straight-chain or branched alkyl, alkoxy, halogen or hydroxyl group
$R^3$ = hydrogen or a straight-chain or branched alkyl group
a=1 to 3
b and c may be 0-2, with the condition that a+b+c = 3 for Me = Si, Ti, Zr and a+b+c = 2 for Me = Al, and
X = a straight-chain or branched alkyl group having up to 20, preferably having 1 to 12 and particularly preferably having 3 to 12, carbon atoms, and
Y = straight-chain or branched alkenyl, alkynyl, alkoxy, SH, $NH_2$, carboxyl, halogen or hydroxyl, where the covalent bond is formed between the organic compound and the spacer group with cleavage or via linking to the reactive group Y, with formation of a covalent bond, and
b) the copolymerisation of the compound obtained in a) with a precursor which is suitable for the construction of an inorganic network which is an oxide, oxide hydrate, phosphate, carbonate, sulfate or nitride, where the additional surface functionalisation takes place by reaction of the resultant particles with the same organic compound present in the interior of the particles and/or organic compounds which are different therefrom selected from the group of the light-absorbent organic compounds, compounds having antioxidant and/or free-radical-inhibiting properties, repellents and preservatives.

**11.** Process according to Claim 10, **characterised in that** step b) is a hydrolytic polycondensation.

**12.** Process according to Claim 10 or 11, **characterised in that** the precursor employed is compounds of the elements magnesium, aluminium, silicon, zinc, cerium, titanium, zirconium, manganese, boron, iron and/or mixtures thereof, which contain hydroxides, halides, alkoxides or other hydrolysable groups.

**13.** Process according to Claim 12, **characterised in that** the precursor is alkoxides of silicon.

**14.** Process according to one of Claims 10 to 13, **characterised in that** the surface of the particles is additionally functionalised with the compounds obtained in accordance with a).

**15.** Use of particles according to Claim 1 in formulations and compositions.

**16.** Compositions comprising particles according to Claim 1 and at least one cosmetically, pharmaceutically and/or dermatologically tolerated vehicle and/or assistant.

**17.** Compositions according to Claim 16, **characterised in that** they are compositions having light-protection properties.

**18.** Compositions according to Claim 16 or 17, **characterised in that** they are compositions which can be applied topically.

**19.** Compositions according to at least one of the preceding claims, **characterised in that** the compositions additionally comprise at least one organic and/or inorganic UV filter.

**20.** Compositions according to at least one of the preceding claims, **characterised in that** the compositions comprise at least one self-tanning agent.

**21.** Compositions according to at least one of the preceding claims, **characterised in that** the compositions comprise at least one photo-stabiliser.

**22.** Compositions according to Claim 21, **characterised in that** the photo-stabiliser is selected from compounds conforming to the formula V

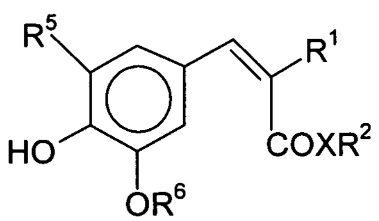

V,

where
$R^1$ is selected from $-C(O)CH_3$, $-CO_2R^3$, $-C(O)NH_2$ and $-C(O)N(R^4)_2$;
X is O or NH;
$R^2$ stands for a linear or branched $C_{1-30}$-alkyl radical;
$R^3$ stands for a linear or branched $C_{1-20}$-alkyl radical,
all $R^4$ independently of one another, stand for H or linear or branched $C_{1-8}$-alkyl radicals
$R^5$ stands for H, a linear or branched $C_{1-8}$-alkyl radical or a linear or branched $-O-C_{1-8}$-alkyl radical and
$R^6$ stands for a $C_{1-8}$-alkyl radical.

**23.** Compositions according to at least one of the preceding claims, **characterised in that** the compositions comprise one or more further UV filters which are selected from the group consisting of 3-(4'-methylbenzylidene)-dl-camphor, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate, 2-phenylbenzimidazole-5-sulfonic acid and potassium, sodium and triethanolamine salts thereof.

**24.** Compositions according to at least one of the preceding claims which are suitable for the protection of body cells against oxidative stress, **characterised in that** they preferably comprise one or more antioxidants.

## Revendications

**1.** Particules comprenant un réseau minéral qui est un oxyde, oxyde hydraté, phosphate, carbonate, sulfate ou nitrure et des composés organiques liés de manière covalente au réseau via un groupement espaceur, **caractérisées en ce que** les composés organiques, choisis parmi le groupe comprenant les dérivés de dibenzoylméthane, l'acide aminobenzoïque, l'acide cinnamique, l'acide salicylique, le benzylidènecamphre, le phénylbenzimidazole, l'acrylate de diphényle, la triazine, la benzophénone, le diarylbutadiène et les amides vinyliques, sont présents à l'intérieur des particules, et la surface des particules a été fonctionnalisée avec le même composé organique présent à l'intérieur des particules et/ou des composés organiques en étant différents choisis parmi le groupe des composés organiques photo-absorbants, des composés ayant des propriétés antioxydantes et/ou anti-radicalaires, des répulsifs et des conservateurs par liaison covalente, et où le groupement espaceur répond à la formule générale

$$(-A)_aMe(R^1)_b(R^2)_c\text{-}X\text{-}$$

dans laquelle
A = O, S, NH,
Me = Si, Al, Ti, Zr,
$R^1$ et $R^2$ peuvent être identiques ou différents et désignent un groupement alkyle, alcoxy, halogène ou hydroxyle à chaîne linéaire ou ramifiée
a va de 1 à 3,
b et c peuvent valoir 0-2, à condition que a+b+c = 3 pour Me = Si, Ti, Zr et a+b+c = 2 pour Me = Al et
X = un groupement alkyle à chaîne linéaire ou ramifiée ayant jusqu'à 20, préférablement ayant de 1 à 12 et particulièrement préférablement ayant de 3 à 12, atomes de carbone, où le groupement espaceur est lié au réseau minéral via le substituant $(-A)_a$.

**2.** Particules selon la revendication 1, **caractérisées en ce qu'**il s'agit de particules sphériques.

**3.** Particules selon la revendication 1 ou 2, **caractérisées en ce que** les particules ont une taille allant de 1 nm à 250 μm.

**4.** Particules selon l'une des revendications 1 à 3, **caractérisées en ce que** l'oxyde est l'oxyde de magnésium, l'oxyde d'aluminium, l'oxyde de silicium, l'oxyde de zinc, l'oxyde de cérium, l'oxyde de titane, l'oxyde de zirconium, l'oxyde de manganèse, l'oxyde de bore, l'oxyde de fer ou un mélange de ceux-ci.

**5.** Particules selon la revendication 4, **caractérisées en ce que** le réseau minéral est l'oxyde de silicium.

**6.** Particules selon l'une des revendications 1 à 5, **caractérisées en ce que** le composé photo-absorbant est choisi parmi le groupe comprenant les dérivés de dibenzoylméthane, l'acide aminobenzoïque, l'acide cinnamique, l'acide salicylique, le benzylidènecamphre, le phénylbenzimidazole, l'acrylate de diphényle, la triazine, la benzophénone, le diarylbutadiène et les amides vinyliques.

**7.** Particules selon l'une des revendications 1 à 5, **caractérisées en ce que** les composés ayant des propriétés antioxydantes et/ou anti-radicalaires sont choisis parmi les flavonoïdes, les coumaranones, les vitamines et le BHT.

**8.** Particules selon l'une des revendications 1 à 5, **caractérisées en ce que** les répulsifs sont choisis parmi les amides et/ou des dérivés de ceux-ci.

**9.** Particules selon l'une des revendications 1 à 5, **caractérisées en ce que** le conservateur est le chlorure de benzalkonium, l'acide benzoïque et les sels de celui-ci, le chlorure de cétylpyridinium, le chlorure de cétrimonium ainsi que l'acide salicylique et les sels de celui-ci.

**10.** Procédé de production de particules selon la revendication 1, comprenant

a) la réaction d'un composé organique choisi parmi le groupe comprenant les dérivés de dibenzoylméthane, l'acide aminobenzoïque, l'acide cinnamique, l'acide salicylique, le benzylidènecamphre, le phénylbenzimidazole, l'acrylate de diphényle, la triazine, la benzophénone, le diarylbutadiène et les amides vinyliques, avec une substance ayant au moins deux groupements réactifs séparés par un groupement espaceur de formule générale

$(R^3A)_aMe(R^1)_b(R^2)_c$-X-Y,

dans laquelle
A = O, S, NH
Me = Si, Al, Ti, Zr
$R^1$ et $R^2$ peuvent être identiques ou différents et désignent un groupement alkyle, alcoxy, halogène ou hydroxyle à chaîne linéaire ou ramifiée
$R^3$ = hydrogène ou un groupement alkyle à chaîne linéaire ou ramifiée a=1à3
b et c peuvent valoir 0-2, à condition que a+b+c = 3 pour Me = Si, Ti, Zr et a+b+c = 2 pour Me = Al, et
X = un groupement alkyle à chaîne linéaire ou ramifiée ayant jusqu'à 20, préférablement ayant de 1 à 12 et particulièrement préférablement ayant de 3 à 12, atomes de carbone, et
Y = alcényle, alkynyle, alcoxy à chaîne linéaire ou ramifiée, SH, $NH_2$, carboxyle, halogène ou hydroxyle, où la liaison covalente est formée entre le composé organique et le groupement espaceur par clivage ou via une liaison au groupement réactif Y, avec formation d'une liaison covalente, et
b) la copolymérisation du composé obtenu en a) avec un précurseur qui est convenable pour la construction d'un réseau minéral qui est un oxyde, oxyde hydraté, phosphate, carbonate, sulfate ou nitrure,

où la fonctionnalisation de surface supplémentaire se produit par réaction des particules résultantes avec le même composé organique présent à l'intérieur des particules et/ou des composés organiques en étant différents choisis parmi le groupe des composés organiques photo-absorbants, des composés ayant des propriétés antioxydantes et/ou anti-radicalaires, des répulsifs et des conservateurs.

**11.** Procédé selon la revendication 10, **caractérisé en ce que** l'étape b) est une polycondensation hydrolytique.

**12.** Procédé selon la revendication 10 ou 11, **caractérisé en ce que** le précurseur employé fait partie des composés des éléments magnésium, aluminium, silicium, zinc, cérium, titane, zirconium, manganèse, bore, fer et/ou des mélanges de ceux-ci, contenant des hydroxydes, des halogénures, des alkylates ou d'autres groupements hydrolysables.

**13.** Procédé selon la revendication 12, **caractérisé en ce que** le précurseur fait partie des alkylates de silicium.

**14.** Procédé selon l'une des revendications 10 à 13, **caractérisé en ce que** la surface des particules est fonctionnalisée en outre par les composés obtenus conformément à a).

**15.** Utilisation des particules selon la revendication 1 dans des formulations et des compositions.

**16.** Compositions comprenant des particules selon la revendication 1 et au moins un véhicule et/ou auxiliaire cosmétiquement, pharmaceutiquement et/ou dermatologiquement toléré.

**17.** Compositions selon la revendication 16, **caractérisées en ce qu'**il s'agit de compositions possédant des propriétés photoprotectrices.

**18.** Compositions selon la revendication 16 ou 17, **caractérisées en ce qu'**il s'agit de compositions qui peuvent être appliquées topiquement.

**19.** Compositions selon au moins l'une des revendications précédentes, **caractérisées en ce que** les compositions comprennent en outre au moins un filtre UV organique et/ou minéral.

**20.** Compositions selon au moins l'une des revendications précédentes, **caractérisées en ce que** les compositions comprennent au moins un agent autobronzant.

**21.** Compositions selon au moins l'une des revendications précédentes, **caractérisées en ce que** les compositions comprennent au moins un photostabilisant.

**22.** Compositions selon la revendication 21, **caractérisées en ce que** le photostabilisant est choisi parmi les composés répondant à la formule V

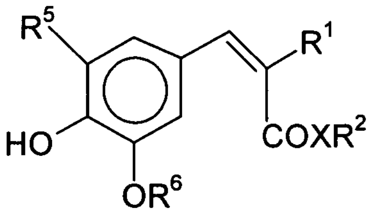

V,

dans laquelle
$R^1$ est choisi parmi $-C(O)CH_3$, $-CO_2R^3$, $-C(O)NH_2$ et $-C(O)N(R^4)_2$ ;
X est O ou NH
$R^2$ représente un radical $C_{1-30}$-alkyle linéaire ou ramifié ;
$R^3$ représente un radical $C_{1-20}$-alkyle linéaire ou ramifié ;
tous les $R^4$ indépendamment les uns des autres, représentent H ou des radicaux $C_{1-8}$-alkyle linéaires ou ramifiés ;
$R^5$ représente H, un radical $C_{1-8}$-alkyle linéaire ou ramifié ou un radical $-O-C_{1-8}$-alkyle linéaire ou ramifié ; et
$R^6$ représente un radical $C_{1-8}$-alkyle.

**23.** Compositions selon au moins l'une des revendications précédentes, **caractérisées en ce que** les compositions comprennent un ou plusieurs filtres UV supplémentaires qui sont choisis parmi le groupe constitué par le 3-(4'-méthylbenzylidène)-dl-camphre, le méthoxycinnamate d'octyle, le salicylate de 3,3,5-triméthylcyclohexyle, le 4-(diméthylamino)benzoate de 2-éthylhexyle, le 2-cyano-3,3-diphénylacrylate de 2-éthylhexyle, l'acide 2-phénylbenzimidazole-5-sulfonique et les sels de potassium, de sodium et de triéthanolamine de ceux-ci.

**24.** Compositions selon au moins l'une des revendications précédentes, qui sont convenables pour la protection de cellules de l'organisme contre un stress oxydatif, **caractérisées en ce qu'**elles contiennent de préférence un ou plusieurs antioxydants.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1205177 A
- WO 9304665 A
- EP 0487404 A
- US 3634588 A
- FR 2326405 A
- FR 2440933 A
- EP 0114607 A
- WO 9966896 A
- US 6242099 B1
- EP 1382328 A
- WO 0009652 A
- WO 0072806 A
- WO 0071084 A
- DE 10244282
- EP 0671161 A
- DE 4116123 A
- WO 03007906 A
- DE OS4308282 A


### In der Beschreibung aufgeführte Nicht-Patentliteratur


- *Römpp Chemie Lexikon,* 1993, vol. 9
- **W. STÖBER et al.** *J. Colloid and Interface Science,* 1968, vol. 26, 62
- *J. COLLOID AND INTERFACE SCIENCE,* 1969, vol. 30, 568
- Rowe Colour Index. Society of Dyers and Colourists. 1971
- **K. Lemanska ; H. Szymusiak ; B. Tyrakowska ; R. Zielinski ; I.M.C.M. Rietjens.** *Current Topics in Biophysics,* 2000, vol. 24 (2), 101-108
- **C.A. Rice-Evans ; N.J. Miller ; G. Paganga.** *Trends in Plant Science,* 1997, vol. 2 (4), 152-159
- **K. Lemanska ; H. Szymusiak ; B. Tyrakowska ; R. Zielinski ; I.M.C:M. Rietjens.** A.E.M.F. Soffers. *Free Radical Biology & Medicine,* 2001, vol. 31 (7), 869-881
- **E. A. Galinski et al.** *Eur. J. Biochem.,* 1985, vol. 149, 135-139